(19) Europäisches Patentamt
European Patent Office
Office européen des brevets

(11) Veröffentlichungsnummer: **0 095 683**
**A1**

(12) # EUROPÄISCHE PATENTANMELDUNG

(21) Anmeldenummer: **83105032.3**

(22) Anmeldetag: **20.05.83**

(51) Int. Cl.³: **C 07 C 131/105**, C 07 D 273/01, C 07 C 131/00, A 01 N 37/52, A 01 N 35/10, C 07 C 161/00 // C07D265/02, C07C119/16, C07C135/00

(30) Priorität: **01.06.82 DE 3220526**

(43) Veröffentlichungstag der Anmeldung: **07.12.83 Patentblatt 83/49**

(84) Benannte Vertragsstaaten: **AT BE CH DE FR GB IT LI**

(71) Anmelder: **BAYER AG, Zentralbereich Patente, Marken und Lizenzen, D-5090 Leverkusen 1, Bayerwerk (DE)**

(72) Erfinder: **Förster, Heinz, Dr., Am Eckbusch 47, D-5600 Wuppertal 1 (DE)**
Erfinder: **Führer, Wolfgang, Dr., Wehrstrasse 28, D-5202 Hennef 1 (DE)**
Erfinder: **Stetter, Jörg, Dr., Gellertweg 4, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch-Gladbach 2 (DE)**
Erfinder: **Lürssen, Klaus, Dr., August-Klerspel-Strasse 89, D-5060 Bergisch-Gladbach 2 (DE)**

(54) **Trifluormethylphenoxy-phenyl-Derivate, Verfahren zu ihrer Herstellung und ihre Verwendung als Herbizide.**

(57) Neue Trifluormethylphenoxy-phenyl-Derivate der Formel

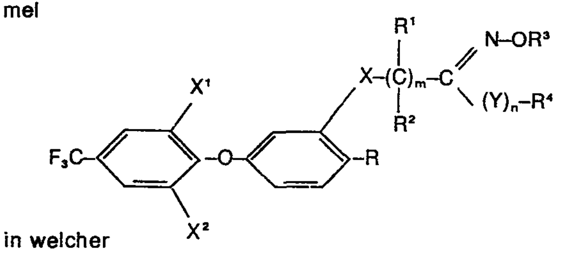

(I)

in welcher

$X^1$ für Wasserstoff, Fluor, Chlor, Brom, Jod, Nitro oder Cyano steht,

$X^2$ für Wasserstoff, Fluor, Chlor, Brom oder Jod steht,

R für Wasserstoff oder Nitro steht,

X für Sauerstoff, eine CO–O-Gruppe oder für die Gruppierung der Formel

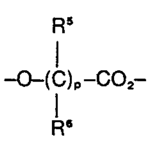

steht,

$R^1$, $R^2$, $R^5$ und $R^6$ unabhängig voneinander für Wasserstoff oder $C_1$–$C_5$-Alkyl stehen,

$R^3$ für Wasserstoff oder $C_1$–$C_5$-Alkyl steht, oder

$R^2$ und $R^3$ gemeinsam für $C_1$–$C_3$-Alkandiyl stehen,

$R^4$ für Wasserstoff oder $C_1$–$C_5$-Alkyl steht, oder

$R^3$ und $R^4$ gemeinsam für $C_1$–$C_3$-Alkandiyl stehen,

Y für Sauerstoff, Schwefel, eine $-NR^7$-gruppe oder eine CO–O-Gruppe steht,

$R^7$ für Wasserstoff oder $C_1$–$C_5$-Alkyl steht,

m für die Zahlen 1, 2 oder 3 steht,

n für die Zahlen 0 oder 1 steht und

p für die Zahlen 1, 2 oder 3 steht,

mehrere Verfahren zur Herstellung der erfindungsgemäßen Stoffe und deren Verwendung als Herbizide.

Neue Ausgangsstoffe zur Herstellung der erfindungsgemäßen Verbindungen und die Synthese dieser neuen Ausgangsstoffe.

**EP 0 095 683 A1**

BAYER AKTIENGESELLSCHAFT 5090 Leverkusen, Bayerwerk

Zentralbereich
Patente, Marken und Lizenzen Dü/ABc
Ib

# Trifluormethylphenoxy-phenyl-Derivate, Verfahren zu ihrer Herstellung und ihre Verwendung als Herbizide

Die Erfindung betrifft neue Trifluormethylphenoxy-phenyl-Derivate, mehrere Verfahren zu ihrer Herstellung sowie ihre Verwendung als Herbizide.

Es ist bereits bekannt, daß bestimmte substituierte Diphenylether herbizide Eigenschaften besitzen (vgl. DE-OS 22 23 894). So kann zum Beispiel der 2-/4-(2,4-Dichlorphenoxy)-phenoxy7-propionsäure-methylester zur Unkrautbekämpfung eingesetzt werden. Die Wirkung dieses Stoffes ist gut, jedoch ist die Selektivität nicht immer ausreichend.

Es wurden nun neue Trifluormethylphenoxy-phenyl-Derivate der Formel

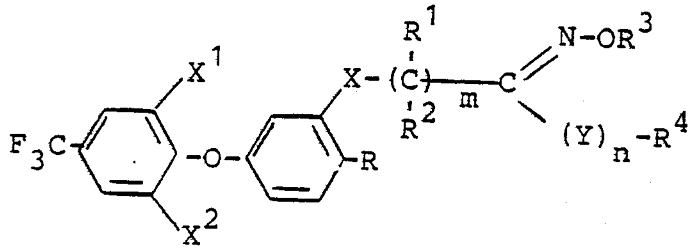

(I)

in welcher

$X^1$ für Wasserstoff, Fluor, Chlor, Brom, Jod, Nitro oder Cyano steht,

$X^2$ für Wasserstoff, Fluor, Chlor, Brom oder Jod steht,

R für Wasserstoff oder Nitro steht,

X für Sauerstoff, eine CO-O-Gruppe oder für die Gruppierung der Formel

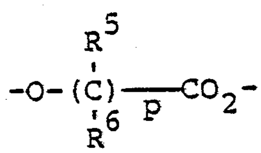

steht,

$R^1$, $R^2$, $R^5$ und $R^6$ unabhängig voneinander für Wasserstoff oder $C_1$-$C_5$-Alkyl stehen,

$R^3$ für Wasserstoff oder $C_1$-$C_5$-Alkyl steht, oder

$R^2$ und $R^3$ gemeinsam für $C_1$-$C_3$-Alkandiyl stehen,

$R^4$ für Wasserstoff oder $C_1$-$C_5$-Alkyl steht, oder

$R^3$ und $R^4$ gemeinsam für $C_1$-$C_3$-Alkandiyl stehen,

Y für Sauerstoff, Schwefel, eine $-NR^7$-Gruppe oder eine CO-O-Gruppe steht,

$R^7$ Wasserstoff oder $C_1$-$C_5$-Alkyl steht,

m für die Zahlen 1, 2 oder 3 steht,

n für die Zahlen 0 oder 1 steht und

p für die Zahlen 1, 2 oder 3 steht,

gefunden.

Die Verbindungen der Formel (I), in denen die Oxim-Einheit nicht Teil eines Ringes ist, können in der syn- oder anti-Form vorliegen. Vorwiegend fallen sie als Gemische beider Formen an. Diejenigen Verbindungen der Formel (I), die eines oder mehrere asymmetrische Kohlenstoffatome enthalten, können in Form von Racematen oder verschiedenen optischen Isomeren auftreten. Die Erfindung betrifft sowohl die sterischen als auch die optischen Isomeren und deren Gemische.

Weiterhin wurde gefunden, daß man neue Trifluormethylphenoxy-phenyl-Derivate der Formel (I) erhält, wenn man

a) Diphenylether der Formel

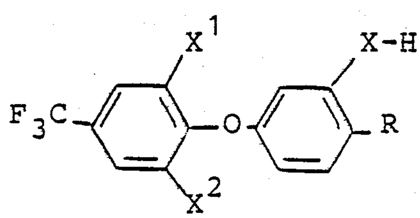

(II)

in welcher

$X^1$, $X^2$, X und R die oben angegebene Bedeutung haben,

mit Verbindungen der Formel

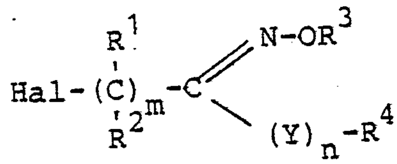

(III)

in welcher

$R^1$, $R^2$, $R^3$, $R^4$, Y, m und n die oben angegebene Bedeutung haben und

Hal für Chlor oder Brom steht,

gegebenenfalls in Gegenwart eines Säureakzeptors und gegegebenenfalls in Gegenwart eines Verdünnungsmittels umsetzt, oder

b) Phenoxyphenole der Formel

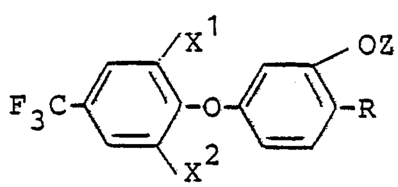

(IV)

in welcher
$X^1$, $X^2$ und R die oben angegebene Bedeutung haben und Z für Wasserstoff oder ein Alkalimetall oder ein Äquivalent eines Erdalkalimetalles steht,

mit Verbindungen der Formel

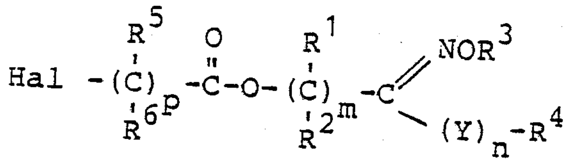

(V)

in welcher

$R^1$, $R^2$, $R^3$, $R^4$, $R^5$, $R^6$, Y, m, n und p die oben angegebene Bedeutung haben und

$Hal^1$ für Chlor oder Brom steht,

gegebenenfalls in Gegenwart eines Säureakzeptors und gegebenenfalls in Gegenwart eines Verdünnungsmittels umsetzt, oder

c) Trifluormethylphenoxy-phenyl-Derivate der Formel

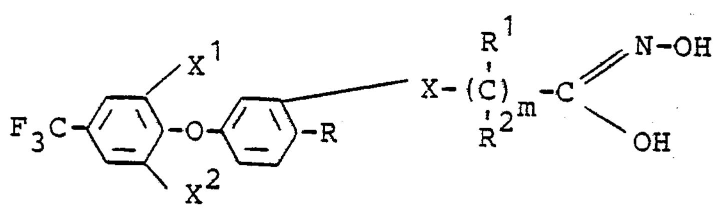

(Ia)

in welcher

$X^1$, $X^2$, X, R, $R^1$, $R^2$ und m die oben angegebene Bedeutung haben, oder deren tautomere Verbindungen, mit Dihalogenalkanen der Formel

$$Hal^2-(CH_2)_q-Hal^2 \qquad (VI)$$

in welcher

q für die Zahlen 1, 2 oder 3 steht und

$Hal^2$ jeweils für Chlor oder Brom steht,

gegebenenfalls in Gegenwart eines Säureakzeptors, und gegebenenfalls in Gegenwart eines Verdünnungsmittels umsetzt, oder

d) Trifluormethylphenoxy-phenyl-Derivate der Formel

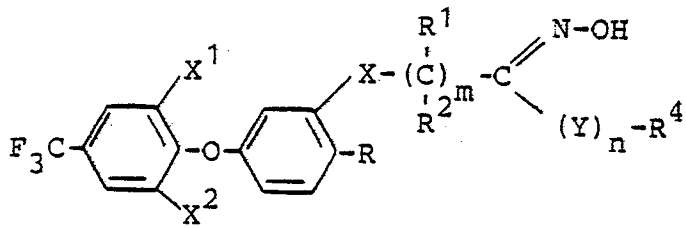

(Ib)

in welcher

$X^1$, $X^2$, X, R, $R^1$, $R^2$, $R^4$, Y, m und n die oben angegebene Bedeutung haben,

mit Alkylierungsmitteln der Formel

$$R^{3'}-Z^1 \qquad \text{(VII)}$$

in welcher

$R^{3'}$ für $C_1$-$C_5$-Alkyl steht, und

$Z^1$ für Chlor, Brom, für eine p-Toluolsulfonyl-Gruppe oder für die Gruppierung $R^8$-O-$SO_2$-O- steht,

worin

$R^8$ für Wasserstoff oder $C_1$-$C_5$-Alkyl steht,

gegebenenfalls in Gegenwart eines Säureakzeptors und gegebenenfalls in Gegenwart eines Verdünnungsmittels umsetzt, oder

e) Halogenide der Formel

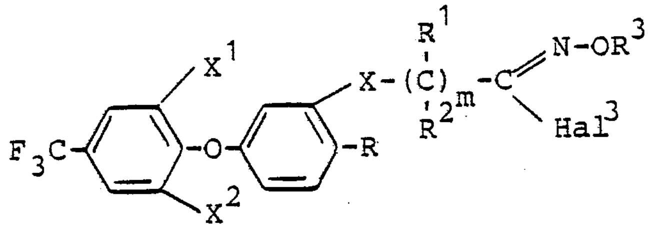

(VIII)

in welcher
$X^1$, $X^2$, X, R, $R^1$, $R^2$, $R^3$ und m die oben angegebene Bedeutung haben und

$Hal^3$ für Chlor oder Brom steht,

mit Verbindungen der Formel

$$R^4\text{-}Q\text{-}Z^2 \quad \text{(IX)}$$

in welcher

$R^4$ die oben angegebene Bedeutung hat,
$Z^2$ für Wasserstoff oder ein Äquivalent eines Alkalimetalles oder eines Erdalkalimetalles steht, und
Q für Sauerstoff, Schwefel oder $NR^7$ steht, worin
$R^7$ die obenangegebene Bedeutung hat,

gegebenenfalls in Gegenwart eines Säureakzeptors und gegebenenfalls in Gegenwart eines Verdünnungsmittels umsetzt, oder

f) Nitrile der Formel

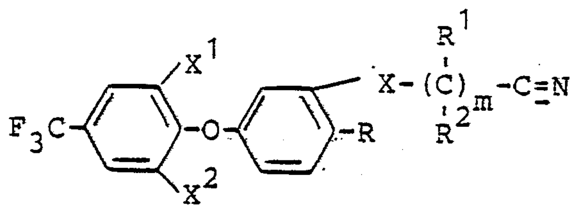

(X)

in welcher

$X^1$, $X^2$, X, R, $R^1$, $R^2$ und m die oben angegebene Bedeutung haben,

mit Hydroxylamin bzw. Hydroxylamin- Hydrochlorid oder -Hydrobromid,

gegebenenfalls in Gegenwart eines Säureakzeptors und gegebenenfalls in Gegenwart eines Verdünnungsmittels umsetzt, oder

g) Verbindungen der Formel

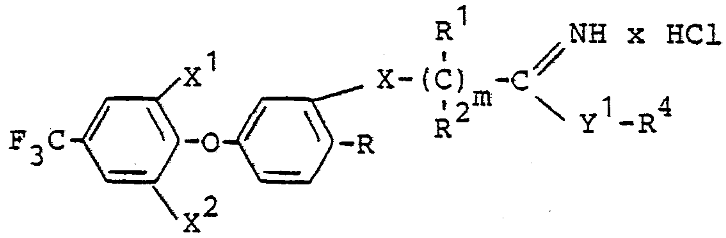

(XI)

in welcher

$X^1$, $X^2$, X, R, $R^1$, $R^2$, $R^4$ und m die oben angegebene Bedeutung haben und

$Y^1$ für Sauerstoff oder Schwefel steht,

mit Hydroxylamin bzw. Hydroxylamin-Hydrochlorid oder -Hydrobromid,

gegebenenfalls in Gegenwart eines Säureakzeptors und gegebenenfalls in Gegenwart eines Verdünnungsmittels umsetzt, oder

h) Trifluormethylphenoxy-phenyl-Derivate der Formel

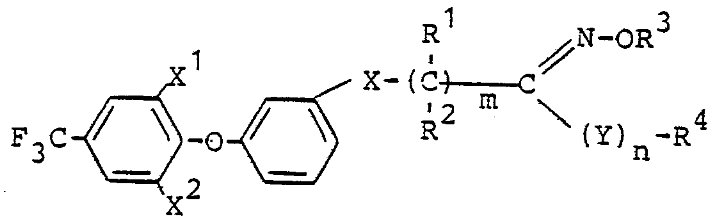

(Ic)

in welcher
X, $X^1$, $X^2$, Y, $R^1$, $R^2$, $R^3$, $R^4$, m und n die oben angegebene Bedeutung haben,

mit Salpetersäure, gegebenenfalls in Gegenwart eines Katalysators sowie gegebenenfalls in Gegenwart eines Verdünnungsmittel umsetzt, oder

i) Carbonyl-Derivate der Formel

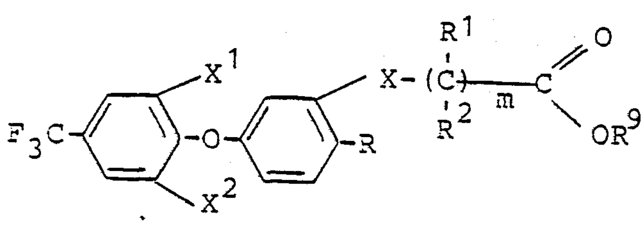

(XII)

in welcher

$X^1$, $X^2$, X, R, $R^1$, $R^2$ und m die oben angegebene Bedeutung haben und

$R^9$ für $C_1$-$C_5$-Alkyl steht,

mit Hydroxylamin oder Hydroxylamin-Hydrochlorid,

gegebenenfalls in Gegenwart eines Säureakzeptors und gegebenenfalls in Gegenwart eines Verdünnungsmittels umsetzt, oder

k) Trifluormethylbenzole der Formel

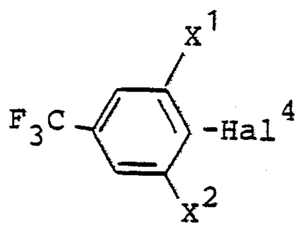

(XIII)

in welcher

$X^1$ und $X^2$ die oben angegebene Bedeutung haben und

$Hal^4$ für Chlor oder Brom steht,

mit Verbindungen der Formel

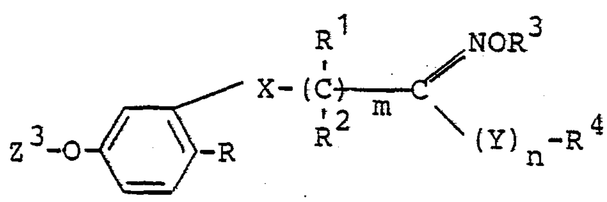

(XIV)

in welcher

X, Y, R, $R^1$, $R^2$, $R^3$, $R^4$, m und n die oben angegebene Bedeutung haben und

$Z^3$ für Wasserstoff, ein Alkalimetall oder ein Äquivalent eines Erdalkalimetalles steht,

gegebenenfalls in Gegenwart eines Säureakzeptors und gegebenenfalls in Gegenwart eines Verdünnungsmittels umsetzt, oder

l) Säurehalogenide der Formel

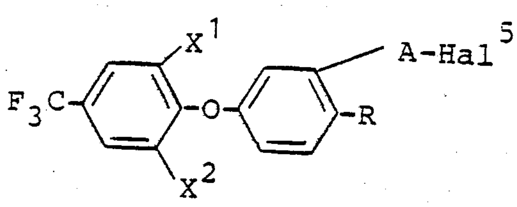

(XV)

in welcher

$X^1$, $X^2$ und R die oben angegebene Bedeutung haben,

$Hal^5$ für Chlor oder Brom steht und

A für eine -CO-Gruppe oder die Gruppierung der Formel

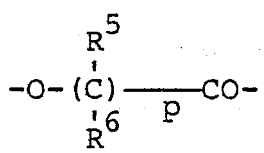

steht,

in welcher

p, $R^5$ und $R^6$ die oben angegebene Bedeutung haben,

mit Verbindungen der Formel

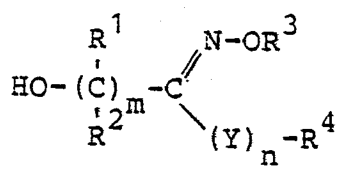

(XVI)

in welcher

$R^1$, $R^2$, $R^3$, $R^4$, Y, m und n die oben angegebene Bedeutung haben,

gegebenenfalls in Gegenwart eines Säureakzeptors und gegebenenfalls in Gegenwart eines Verdünnungsmittels umsetzt.

Schließlich wurde gefunden, daß sich die neuen Trifluormethyl-phenoxy-phenyl-Derivate der Formel (I) durch hervorragende herbizide Eigenschaften auszeichnen.

Überraschenderweise besitzen die erfindungsgemäßen Trifluormethylphenoxy-phenyl-Derivate der Formel (I) eine wesentlich bessere herbizide Wirksamkeit als der 2-/4-(2,4-Dichlorphenoxy)-phenoxy/-propionsäure-methylester, welcher ein konstitutionell ähnlicher vorbekannter Wirkstoff gleicher Wirkungsrichtung ist. Die erfindungsgemäßen Verbindungen lassen sich außerdem unerwarteterweise sehr gut zur Entlaubung von Pflanzen einsetzen.

Die erfindungsgemäßen Trifluormethylphenoxy-phenyl-Derivate sind durch die Formel (I) allgemein definiert.

In dieser Formel stehen vorzugsweise

$X^1$ für Wasserstoff, Fluor, Chlor, Nitro oder Cyano,

$X^2$ für Wasserstoff, Fluor oder Chlor,

R für Wasserstoff oder Nitro,

X für Sauerstoff, eine -COO-Gruppe oder für die Gruppierung der Formel

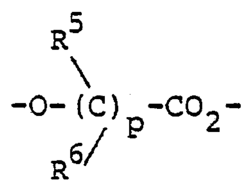

$R^1$, $R^2$, $R^5$ und $R^6$ unabhängig voneinander für Wasserstoff, Methyl oder Ethyl,

$R^3$ für Wasserstoff, Methyl, Ethyl, n-Propyl oder iso-Propyl, oder

$R^2$ und $R^3$ gemeinsam für Ethandiyl,

$R^4$ für Wasserstoff, Methyl, Ethyl, n-Propyl oder iso-Propyl,

oder

$R^3$ und $R^4$ gemeinsam für Ethandiyl,

Y für Sauerstoff, Schwefel, eine $-NR^7$-Gruppe oder eine -CO-O-Gruppe,

$R^7$ für Wasserstoff, Methyl, Ethyl, n-Propyl oder iso-Propyl,

m für die Zahl 1,

n für die Zahlen 0 oder 1 und

p für die Zahl 1.

Bevorzugt sind außerdem die entsprechenden geometrischen und optischen Isomeren.

Besonders bevorzugte optische Isomere sind dabei die R- bzw. S-Enantiomeren der Verbindungen der Formeln

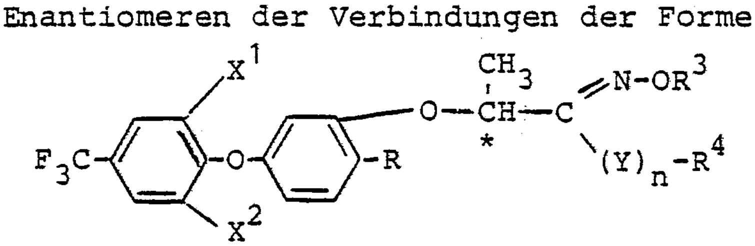

(Id)

und

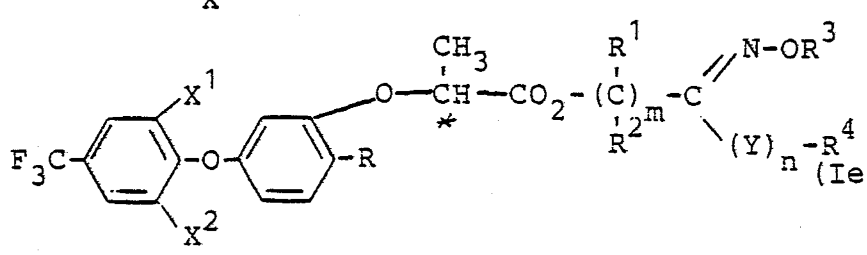

(Ie)

in welchen
$X^1$, $X^2$, $R^1$, $R^2$, $R^3$, $R^4$, R, Y, m und n die oben angegebenen vorzugsweisen Bedeutungen haben und in denen das mit dem Sauerstoffatom verbundene, durch (*) gekennzeichnete Kohlenstoffatom in der Seitenkette ein Chiralitätszentrum darstellt.

Als Beispiele für die erfindungsgemäßen Stoffe der Formeln (I) seien im einzelnen die in den folgenden Tabellen 1 und 2 formelmäßig aufgeführten Stoffe genannt.

Tabelle 1

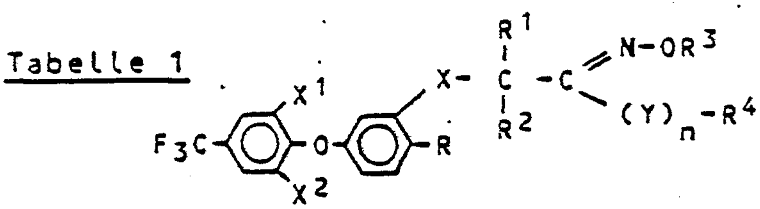

(If)

| X | $X^1$ | $X^2$ | R | $R^1$ | $R^2$ | $R^3$ | $R^4$ | Y | n |
|---|---|---|---|---|---|---|---|---|---|
| O | Cl | H | H | H | $-CH_3$ | $-CH_2-CH_2-$ | | O | 1 |
| O | H | H | H | H | $-CH_3$ | $-CH_2-CH_2-$ | | O | 1 |
| O | Cl | Cl | H | H | $-CH_3$ | $-CH_2-CH_2-$ | | O | 1 |
| O | H | H | $-NO_2$ | H | $-CH_3$ | $-CH_2-CH_2-$ | | O | 1 |
| O | Cl | H | H | H | $-CH_3$ | $-C_2H_5$ | $-C_2H_5$ | O | 1 |
| O | H | H | H | H | $-CH_3$ | $-C_2H_5$ | $-C_2H_5$ | O | 1 |
| O | Cl | Cl | H | H | $-CH_3$ | $-C_2H_5$ | $-C_2H_5$ | O | 1 |
| O | H | H | $-NO_2$ | H | $-CH_3$ | $-C_2H_5$ | $-C_2H_5$ | O | 1 |
| O | Cl | Cl | $-NO_2$ | H | $-CH_3$ | $-C_2H_5$ | $-C_2H_5$ | O | 1 |
| O | Cl | H | H | H | $-CH_3$ | $-CH_3$ | $-C_2H_5$ | O | 1 |
| O | H | H | H | H | $-CH_3$ | $-CH_3$ | $-C_2H_5$ | O | 1 |
| O | Cl | Cl | H | H | $-CH_3$ | $-CH_3$ | $-C_2H_5$ | O | 1 |
| O | H | H | $-NO_2$ | H | $-CH_3$ | $-CH_3$ | $C_2H_5$ | O | 1 |
| O | Cl | Cl | $-NO_2$ | H | $-CH_3$ | $-CH_3$ | $-C_2H_5$ | O | 1 |
| O | Cl | H | H | H | $-CH_3$ | $-CH_3$ | $-CH_3$ | S | 1 |
| O | H | H | H | H | $-CH_3$ | $-CH_3$ | $-CH_3$ | S | 1 |
| O | H | H | $-NO_2$ | H | $-CH_3$ | $-CH_3$ | $-CH_3$ | S | 1 |

Tabelle 1 (Fortsetzung)

| X | $X^1$ | $X^2$ | R | $R^1$ | $R^2$ | $R^3$ | $R^4$ | Y | n |
|---|---|---|---|---|---|---|---|---|---|
| O | Cl | H | $-NO_2$ | H | $-CH_3$ | $-C_3H_7$-n | $-C_3H_7$-n | O | 1 |
| O | H | H | $-NO_2$ | H | $-CH_3$ | $-C_3H_7$-n | $-C_3H_7$-n | O | 1 |
| O | Cl | H | H | H | $-CH_3$ | $-C_3H_7$-n | $-C_3H_7$-n | O | 1 |
| O | Cl | Cl | $-NO_2$ | H | $-CH_3$ | $-C_3H_7$-n | $-C_3H_7$-n | O | 1 |
| O | Cl | Cl | H | H | $-CH_3$ | $-C_3H_7$-n | $-C_3H_7$-n | O | 1 |
| O | H | H | H | H | $-CH_3$ | $-C_3H_7$-n | $-C_3H_7$-n | O | 1 |
| O | H | H | $-NO_2$ | H | $-CH_3$ | $-C_3H_7$-i | $-CH_3$ | O | 1 |
| O | Cl | H | H | H | $-CH_3$ | $-C_3H_7$-i | $-CH_3$ | O | 1 |
| O | Cl | Cl | H | H | $-CH_3$ | $C_3H_7$-i | $-CH_3$ | O | 1 |
| O | Cl | Cl | $-NO_2$ | H | $-CH_3$ | $-C_3H_7$-i | $-CH_3$ | O | 1 |
| O | H | H | $-NO_2$ | H | $-CH_3$ | $-C_3H_7$-i | $-CH_3$ | O | 1 |
| O | H | H | H | H | $-CH_3$ | $-C_3H_7$-i | $-CH_3$ | O | 1 |
| O | Cl | H | $-NO_2$ | H | $-CH_3$ | $-C_3H_7$-i | $-CH_3$ | O | 1 |
| O | Cl | H | $-NO_2$ | H | -H | $-C_2H_5$ | $-C_2H_5$ | O | 1 |
| O | Cl | Cl | $-NO_2$ | H | H | $-C_2H_5$ | $-C_2H_5$ | O | 1 |
| O | Cl | Cl | H | H | H | $-C_2H_5$ | $-C_2H_5$ | O | 1 |
| O | H | H | H | H | H | $-C_2H_5$ | $-C_2H_5$ | O | 1 |
| O | Cl | H | H | H | H | $-C_2H_5$ | $-C_2H_5$ | O | 1 |
| O | H | H | $-NO_2$ | H | H | $-C_2H_5$ | $-C_2H_5$ | O | 1 |

Tabelle 1 (Fortsetzung)

| X | $X^1$ | $X^2$ | R | $R^1$ | $R^2$ | $R^3$ | $R^4$ | Y | n |
|---|---|---|---|---|---|---|---|---|---|
| O | Cl | Cl | H | H | $-CH_3$ | $-C_2H_5$ | H | - | 0 |
| O | Cl | H | H | H | $-CH_3$ | $-C_2H_5$ | H | - | 0 |
| O | H | H | $-NO_2$ | H | $-CH_3$ | $-C_2H_5$ | H | - | 0 |
| O | H | H | H | H | $-CH_3$ | $-C_2H_5$ | H | - | 0 |
| O | Cl | Cl | H | H | $-CH_3$ | $-CH_3$ | H | - | 0 |
| O | Cl | H | H | H | $-CH_3$ | $-CH_3$ | H | - | 0 |
| O | H | H | $-NO_2$ | H | $-CH_3$ | $-CH_3$ | H | - | 0 |
| O | H | H | H | H | $-CH_3$ | $-CH_3$ | H | - | 0 |
| O | Cl | Cl | H | H | $-CH_3$ | $-C_2H_5$ | H | - | 0 |
| O | Cl | H | H | H | $-CH_3$ | $-C_2H_5$ | H | - | 0 |
| O | H | H | $-NO_2$ | H | $-CH_3$ | $-C_2H_5$ | H | - | 0 |
| O | H | H | H | H | $-CH_3$ | $-C_2H_5$ | H | - | 0 |

Tabelle 1 (Fortsetzung)

| X | $X^1$ | $X^2$ | R | $R^1$ | $R^2$ | $R^3$ | $R^4$ | Y | n |
|---|---|---|---|---|---|---|---|---|---|
| O | Cl | Cl | H | H | H | $-CH_3$ | H | - | 0 |
| O | Cl | H | $-NO_2$ | H | H | $-CH_3$ | H | - | 0 |
| O | H | H | $-NO_2$ | H | H | $-CH_3$ | H | - | 0 |
| O | H | H | H | H | H | $-CH_3$ | H | - | 0 |
| O | Cl | H | H | H | H | $-CH_3$ | H | - | 0 |
| O | Cl | H | H | H | $-CH_2$ | $-CH_2-$ | $-C_2H_5$ | O | 1 |
| O | H | H | $-NO_2$ | H | $-CH_2$ | $-CH_2-$ | $-C_2H_5$ | O | 1 |
| O | H | H | H | H | $-CH_2$ | $-CH_2-$ | $-C_2H_5$ | O | 1 |
| O | H | H | $-NO_2$ | $-CH_3$ | $-CH_3$ | $-CH_3$ | H | - | 0 |
| O | Cl | H | $-NO_2$ | $-CH_3$ | $-CH_3$ | $-CH_3$ | H | - | 0 |
| O | Cl | Cl | $-NO_2$ | $-CH_3$ | $-CH_3$ | $-CH_3$ | H | - | 0 |
| O | H | H | H | $-CH_3$ | $-CH_3$ | $-CH_3$ | H | - | 0 |
| O | Cl | H | H | $-CH_3$ | $-CH_3$ | $-CH_3$ | H | - | 0 |
| O | Cl | Cl | H | $-CH_3$ | $-CH_3$ | $-CH_3$ | H | - | 0 |
| O | H | H | $-NO_2$ | H | H | $-CH_2$ | $-CH_2-$ | O | 1 |
| O | Cl | H | $-NO_2$ | H | H | $-CH_2$ | $-CH_2-$ | O | 1 |
| O | Cl | Cl | $-NO_2$ | H | H | $-CH_2$ | $-CH_2-$ | O | 1 |
| O | H | H | H | H | H | $-CH_2$ | $-CH_2-$ | O | 1 |
| O | Cl | H | H | H | H | $-CH_2$ | $-CH_2-$ | O | 1 |
| O | Cl | Cl | H | H | H | $-CH_2$ | $-CH_2-$ | O | 1 |
| O | $-NO_2$ | H | H | H | H | $-CH_2-$ | $CH_2-$ | O | 1 |
| O | $-NO_2$ | H | H | H | H | $-CH_3$ | $-CH_3$ | O | 1 |
| O | $-NO_2$ | H | H | H | H | $-C_2H_5$ | $-CH_3$ | S | 1 |
| O | $-NO_2$ | H | H | H | H | $-C_2H_5$ | $-CH_3$ | S | 1 |

Tabelle 1 (Fortsetzung)

| X | $X^1$ | $X^2$ | R | $R^1$ | $R^2$ | $R^3$ | $R^4$ | Y | n |
|---|---|---|---|---|---|---|---|---|---|
| O | Cl | H | $-NO_2$ | H | $-CH_3$ | $-CH_3$ | $-C_2H_5$ | $-CO_2$ | 1 |
| O | H | H | $-NO_2$ | H | $-CH_3$ | $-CH_3$ | $-C_2H_5$ | $-CO_2$ | 1 |
| O | Cl | Cl | $-NO_2$ | H | $-CH_3$ | $-CH_3$ | $-C_2H_5$ | $-CO_2$ | 1 |
| O | Cl | H | H | H | $-CH_3$ | $-CH_3$ | $-C_2H_5$ | $-CO_2$ | 1 |
| O | H | H | H | H | $-CH_3$ | $-CH_3$ | $-C_2H_5$ | $-CO_2$ | 1 |
| $-CO_2$ | Cl | Cl | $-NO_2$ | H | $-CH_3$ | $-CH_3$ | H | - | 0 |
| $-CO_2$ | Cl | Cl | H | H | $-CH_3$ | $-CH_3$ | H | - | 0 |
| $-CO_2$ | Cl | H | H | H | $-CH_3$ | $-CH_3$ | H | - | 0 |
| $-CO_2$ | H | H | $-NO_2$ | H | $-CH_3$ | $-CH_3$ | H | - | 0 |
| $-CO_2$ | H | H | H | H | $-CH_3$ | $-CH_3$ | H | - | 0 |
| $-CO_2$ | H | H | $-NO_2$ | H | $-CH_3$ | $-C_2H_5$ | H | - | 0 |
| $-CO_2$ | Cl | H | H | H | $-CH_3$ | $-C_2H_5$ | H | - | 0 |
| $-CO_2$ | Cl | Cl | H | H | $-CH_3$ | $-C_2H_5$ | H | - | 0 |
| $-CO_2$ | H | H | H | H | $-CH_3$ | $-C_2H_5$ | H | - | 0 |
| $-CO_2$ | H | H | $-NO_2$ | H | H | $-CH_3$ | H | - | 0 |
| $-CO_2$ | H | H | H | H | H | $-CH_3$ | H | - | 0 |
| $-CO_2$ | Cl | H | H | H | H | $-CH_3$ | H | - | 0 |
| $-CO_2$ | Cl | Cl | H | H | H | $-CH_3$ | H | - | 0 |
| $-CO_2$ | H | H | $-NO_2$ | $-CH_3$ | $-CH_3$ | $-CH_3$ | H | - | 0 |
| $-CO_2$ | Cl | H | $-NO_2$ | $-CH_3$ | $-CH_3$ | $-CH_3$ | H | - | 0 |

Tabelle 1 (Fortsetzung)

| X | $X^1$ | $X^2$ | R | $R^1$ | $R^2$ | $R^3$ | $R^4$ | Y | n |
|---|---|---|---|---|---|---|---|---|---|
| $-CO_2$ | Cl | Cl | $-NO_2$ | $-CH_3$ | $-CH_3$ | $-CH_3$ | H | - | 0 |
| $-CO_2$ | Cl | Cl | H | $-CH_3$ | $-CH_3$ | $-CH_3$ | H | - | 0 |
| $-CO_2$ | Cl | H | H | $-CH_3$ | $-CH_3$ | $-CH_3$ | H | - | 0 |
| $-CO_2$ | H | H | H | $-CH_3$ | $-CH_3$ | $-CH_3$ | H | - | 0 |

Tabelle 2

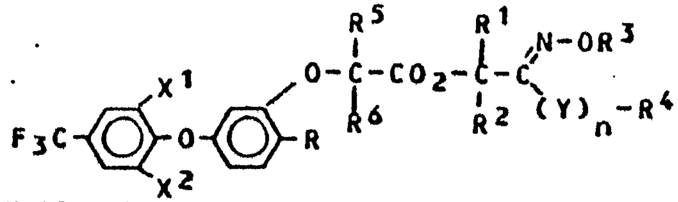

(Ig)

| $X^1$ | $X^2$ | R | $R^1$ | $R^2$ | $R^3$ | $R^4$ | $R^5$ | $R^6$ | Y | n |
|---|---|---|---|---|---|---|---|---|---|---|
| Cl | Cl | $-NO_2$ | H | $-CH_3$ | $-CH_2-CH_2$ | | $-CH_3$ | H | O | 1 |
| Cl | H | $-NO_2$ | H | $-CH_3$ | $-CH_2-CH_2$ | | $-CH_3$ | H | O | 1 |
| H | H | $-NO_2$ | H | $-CH_3$ | $-CH_2-CH_2$ | | $-CH_3$ | H | O | 1 |
| Cl | H | H | H | $-CH_3$ | $-CH_2-CH_2$ | | $-CH_3$ | H | O | 1 |
| H | H | H | H | $-CH_3$ | $-CH_2-CH_2$ | | $-CH_3$ | H | O | 1 |
| Cl | H | $-NO_2$ | $-CH_3$ | $-CH_3$ | $-CH_3$ | H | $-CH_3$ | H | – | 0 |
| Cl | Cl | $-NO_2$ | $-CH_3$ | $-CH_3$ | $-CH_3$ | H | $-CH_3$ | H | – | 0 |
| H | H | $-NO_2$ | $-CH_3$ | $-CH_3$ | $-CH_3$ | H | $-CH_3$ | H | – | 0 |
| H | H | H | $-CH_3$ | $-CH_3$ | $-CH_3$ | H | $-CH_3$ | H | – | 0 |
| Cl | Cl | H | $-CH_3$ | $-CH_3$ | $-CH_3$ | H | $-CH_3$ | H | – | 0 |
| Cl | H | H | $-CH_3$ | $-CH_3$ | $-CH_3$ | H | $-CH_3$ | H | – | 0 |

Verwendet man 5-(2,6-Dichlor-4-trifluormethyl-phenoxy)-2-nitro-benzoesäure und 2-Brom-1-methoximino-propan als Ausgangsstoffe, so kann der Verlauf des erfindungsgemäßen Verfahrens (a) durch das folgende Formelschema wiedergegeben werden:

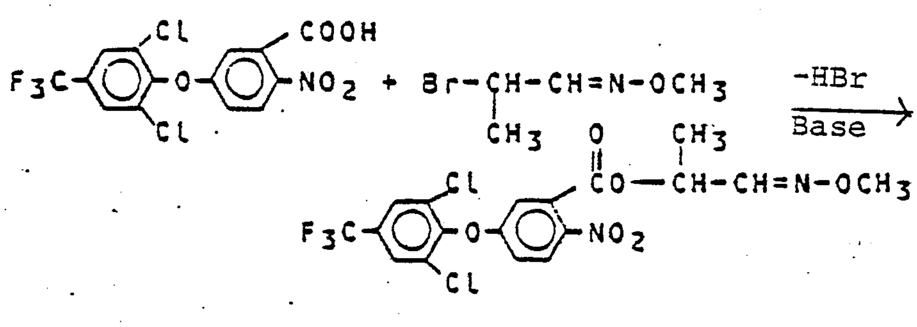


Verwendet man 5-(2-Chlor-4-trifluormethyl-phenoxy)-2-nitro-phenol und Chloressigsäure-2-(1-methoximino)-propylester als Ausgangsstoffe, so kann der Verlauf des erfindungsgemäßen Verfahrens (b) durch das folgende Formelschema wiedergegeben werden:

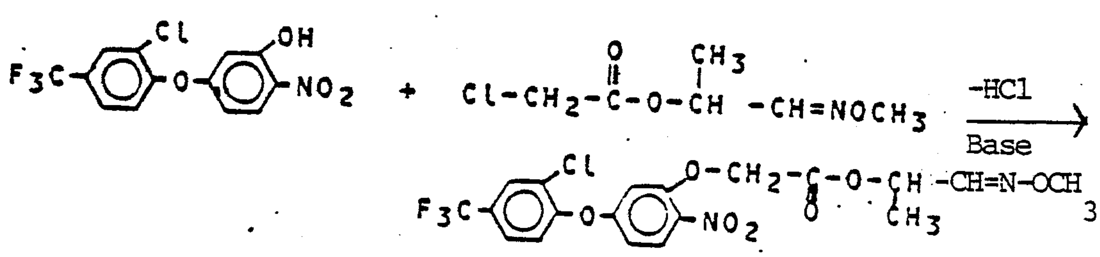

Verwendet man 2-[3-(2,6-Dichlor-4-trifluormethyl-phenoxy)-phenoxy]-N-hydroxy-propionamid und 1,2-Dibromethan als Ausgangsstoffe, so kann der Verlauf des erfindungsgemäßen Verfahrens (c) durch das folgende Formelschema wiedergegeben werden:

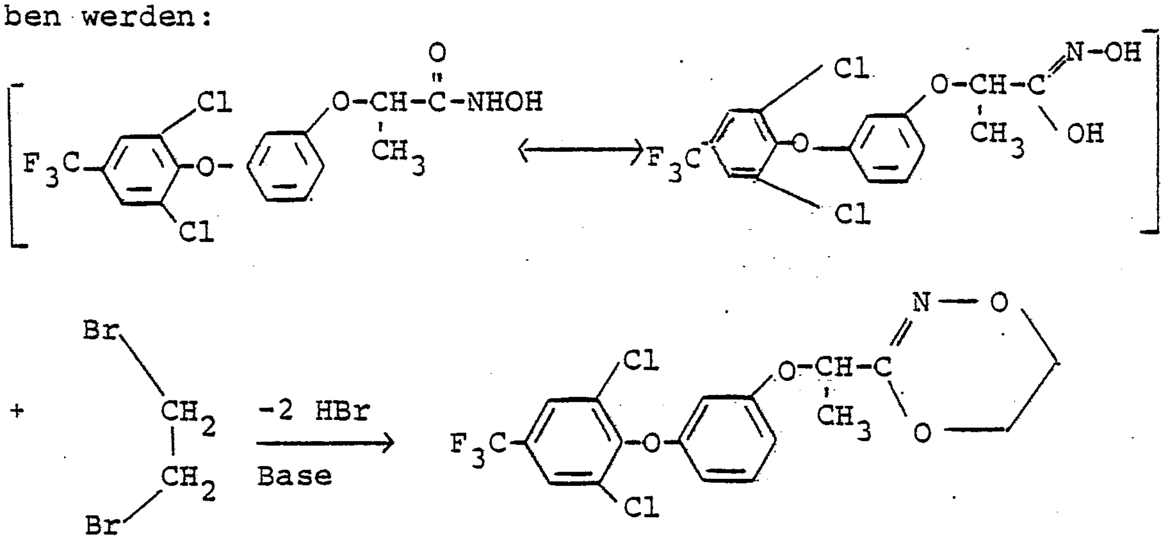


Verwendet man 2-[3-(2-Chlor-4-trifluormethyl-phenoxy)-phenoxy]-1-hydroximino-1-methoxy-propan und Dimethylsulfat als Ausgangsstoffe, so kann der Verlauf des erfindungsgemäßen Verfahrens (d) durch das folgende Formelschema wiedergegeben werden:

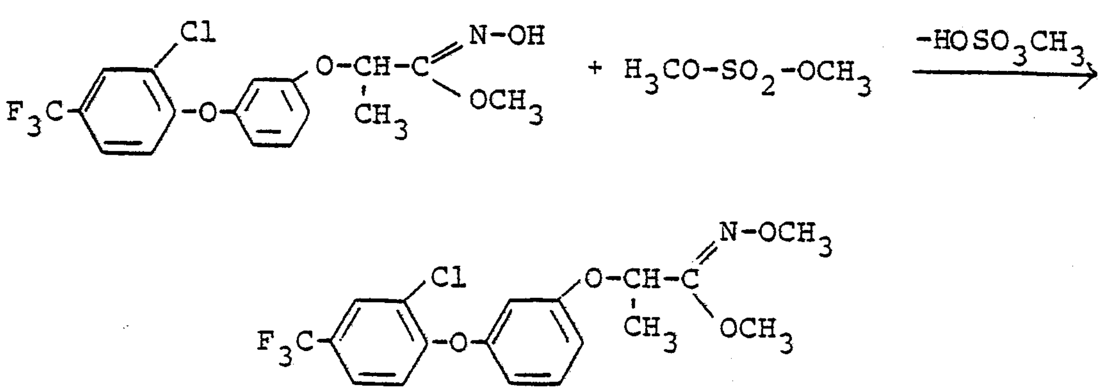

Verwendet man 1-Chlor-2-/3-(4-trifluormethyl-phenoxy)-phenoxy7-1-methoximino-ethan und Natriumethanolat als Ausgangsstoffe, so kann der Verlauf des erfindungsgemäßen Verfahrens (e) durch das folgende Formelschema wiedergegeben werden:

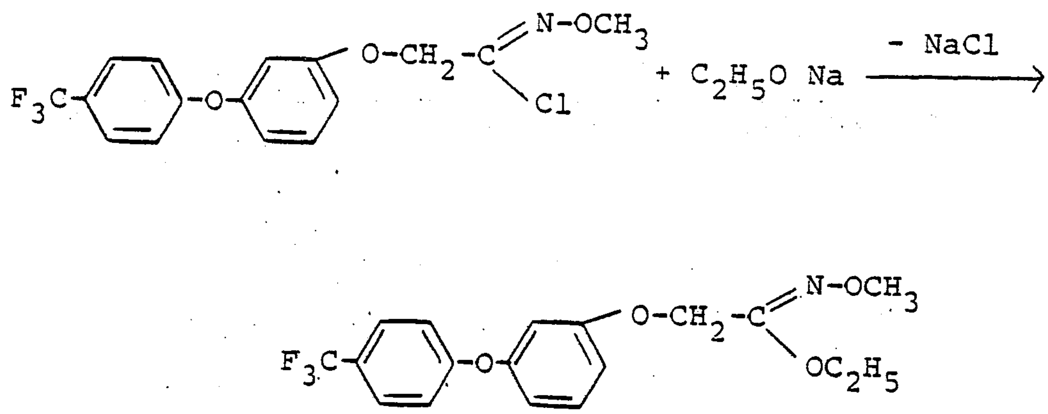


Verwendet man 2-/3-(2-Chlor-4-trifluormethyl-phenoxy)-phenoxy7-acetonitril und Hydroxylamin als Ausgangsstoffe so kann der Verlauf des erfindungsgemäßen Verfahrens (f) durch das folgende Formelschema wiedergegeben werden:

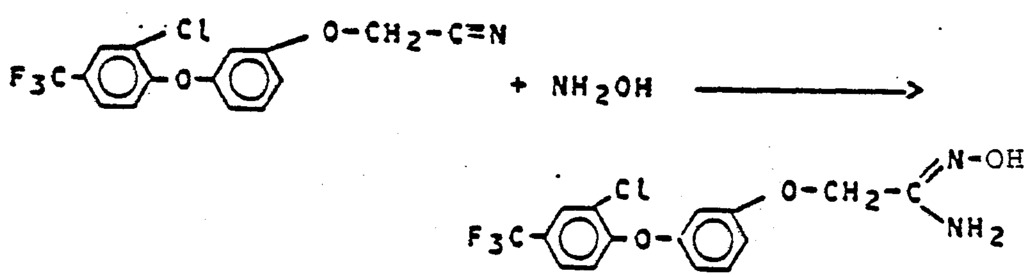

Verwendet man das Hydrochlorid des 2-[3-(2-Chlor-4-trifluormethyl-phenoxy)-phenoxy]-1-imido-essigsäureethylesters und Hydroxylamin als Ausgangsstoffe, so kann der Verlauf des erfindungsgemäßen Verfahrens (g) durch das folgende Formelschema wiedergegeben werden:

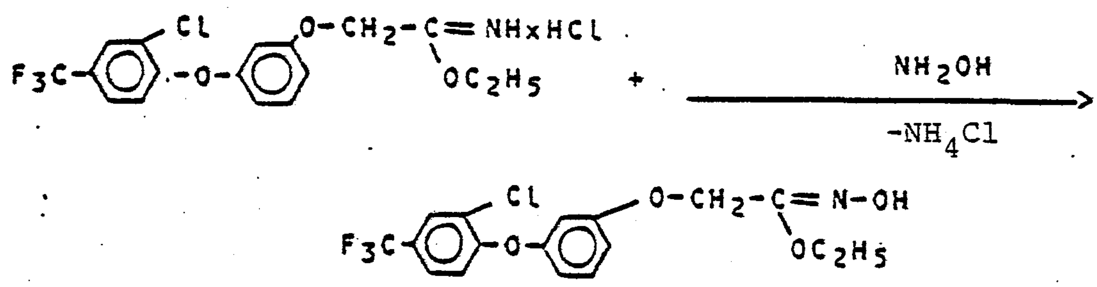


Verwendet man 3-(2,6-Dichlor-4-trifluormethyl-phenoxy)-benzoesäure-1-(1,4-diox-2-azin-3-yl)-ethylester und Salpetersäure als Ausgangsstoffe, so kann der Verlauf des erfindungsgemäßen Verfahrens (h) durch das folgende Formelschema wiedergegeben werden:

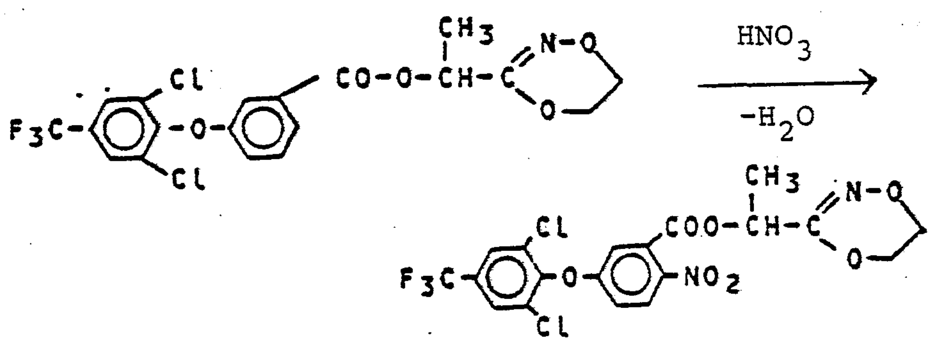

Verwendet man 2-/3-(4-Trifluormethyl-phenoxy)-phenoxy7-propionsäureethylester und Hydroxylamin-Hydrochlorid als Ausgangsstoffe, so kann der Verlauf des erfindungsgemäßen Verfahrens (i) durch das folgende Formelschema wiedergegeben werden:

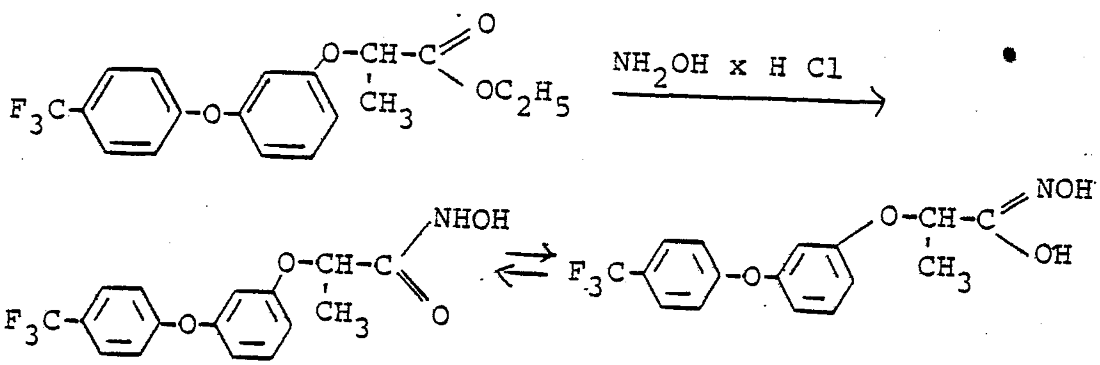


Verwendet man 1,2,3-Trichlor-5-trifluormethyl-benzol und 1-(3-Hydroxy-phenoxy)-1-(1,4-diox-2-azin-3-yl)-ethan als Ausgangsstoffe, so kann der Verlauf des erfindungsgemäßen Verfahrens (k) durch das folgende Formelschema wiedergegeben werden:

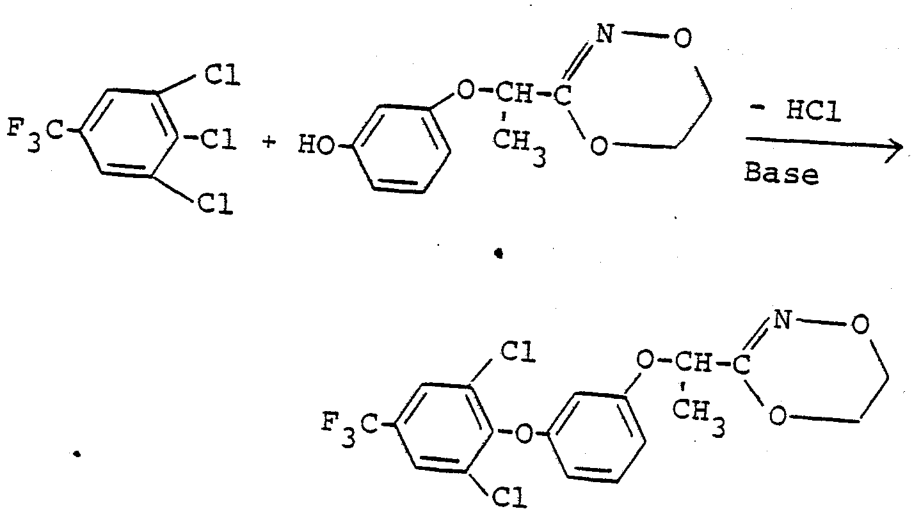


Verwendet man 2-/3-(4-Trifluormethylphenoxy)-phenoxy7-

propionsäurechlorid und 1-Methoximino-2-propanol als Ausgangsstoffe, so kann der Verlauf des erfindungsgemäßen Verfahrens (1) durch das folgende Formelschema wiedergegeben werden:

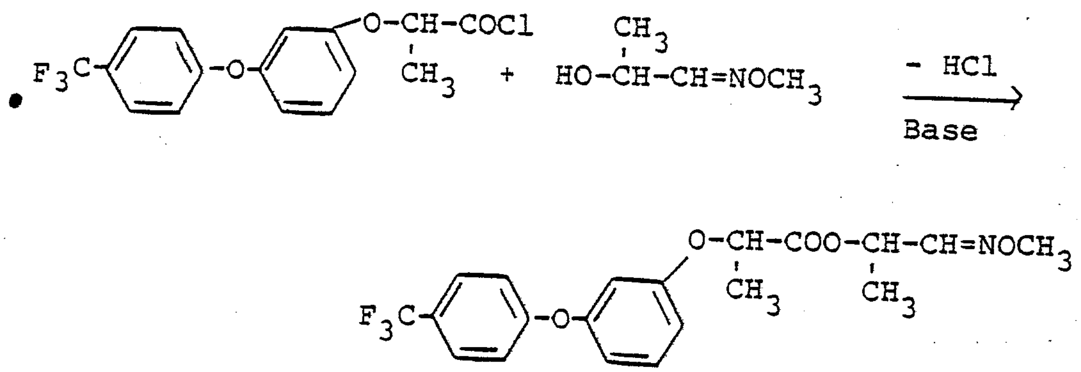


Die bei der Durchführung des erfindungsgemäßen Verfahrens (a) als Ausgangsstoffe benötigten Diphenylether sind durch die Formel (II) definiert. In dieser Formel haben $X^1$, $X^2$, X und R vorzugsweise diejenigen Bedeutungen, die bereits in Zusammenhang mit der Beschreibung der erfindungsgemäßen Stoffe der Formel (I) für diese Reste vorzugsweise genannt wurden.

Die Verbindungen der Formel (II) sind bereits bekannt oder lassen sich nach üblichen Methoden in einfacher Weise herstellen (vergl. DE-OS 2 311 638, GB-PS 2 058 055 und US-PS 4 031 131).

Die weiterhin bei dem erfindungsgemäßen Verfahren (a) als Ausgangsstoffe benötigten Verbindungen sind durch

die Formel (III) definiert. In dieser Formel haben $R^1$, $R^2$, $R^3$, $R^4$, Y, m und n vorzugsweise diejenigen Bedeutungen, die bereits in Zusammenhang mit der Beschreibung der erfindungsgemäßen Stoffe der Formel (I) vorzugsweise für diese Reste bzw. Indices genannt wurden. Hal steht für Chlor oder Brom.

Die Verbindungen der Formel (III) sind bekannt (vgl.

US-PS 3 896 189, DE-OS 29 22 759, DE-OS 31 00 728 und DE-OS 19 37 454) oder lassen sich nach üblichen Methoden in einfacher Weise herstellen.

So erhält man Verbindungen der Formel (III) z.B. dadurch, daß man

- die zugrunde liegenden Carbonylverbindungen mit Hydroxylamin bzw. Hydroxylamin-Derivaten in Gegenwart eines Lösungsmittels, wie z.B. eines Alkohols, bei Temperaturen zwischen 0°C und 100°C, vorzugsweise zwischen 0°C und 80°C umsetzt. Das Hydroxylamin bzw. Hydroxylamin-Derivat wird dabei vorzugsweise in Form eines Salzes, insbesondere als Hydrochlorid, gegebenenfalls in Gegenwart eines Säurebindemittels, wie beispielsweise Natriumacetat, eingesetzt.

Außerdem lassen sich Verbindungen der Formel (III) auch dadurch herstellen, daß man Verbindungen der Formel

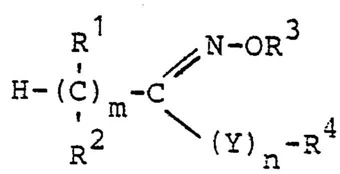

(XVII)

in welcher

$R^1$, $R^2$, $R^3$, $R^4$, Y, m und n die oben angegebene Bedeutung haben,

in üblicher Weise chloriert oder bromiert (siehe hierzu die Herstellungsbeispiele);

oder indem man Alkohole der Formel

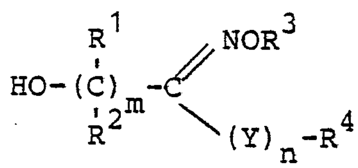

(XVI)

in welcher

$R^1$, $R^2$, $R^3$, $R^4$, Y, m und n die oben angegebene Bedeutung haben,

mit Halogenierungsmitteln, wie Thionylchlorid oder Thionylbromid, gegebenenfalls in Gegenwart eines Säureakzeptors und gegebenenfalls in Gegenwart eines Verdünnungsmittels umsetzt.

Bei der Durchführung des erfindungsgemäßen Verfahrens (a) arbeitet man vorzugsweise in Gegenwart eines Säureakzeptors. Als solche können alle üblichen Säurebindemittel Verwendung finden. Vorzugsweise in Frage kommen Alkali-hydroxide, wie z.B. Natrium- und Kalium-hydroxid, Alkalicarbonate und -alkoholate, wie Natrium- und

Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Diazabicyclooctan, 1,5-Diazabicyclo[4,3,0]-non-5-en und 1,8-Diazabicyclo[5,4,0]undec-7-en.

Als Verdünnungsmittel können bei der Durchführung des erfindungsgemäßen Verfahrens (a) praktisch alle inerten organischen Solventien verwendet werden. Vorzugsweise in Betracht kommen aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, ferner Ether, wie Diethylether und Dibutylether, Glycoldimethylether und Diglycoldimethylether, Tetrahydrofuran und Dioxan, außerdem Ketone, wie Aceton, Methylethyl-, Methyl-isopropyl- und Methyl-isobutylketon, Ester wie Essigsäure-methylester und -ethylester, weiterhin Nitrile, z.B. Acetonitril und Propionitril, darüber hinaus Amide, wie z.B. Dimethylformamid, Dimethylacetamid und N-Methyl-pyrrolidon, sowie stark polare Solventien wie Dimethylsulfoxid, Tetramethylensulfon und Hexamethylphosphorsäuretriamid.

Die Reaktionstemperaturen können bei der Durchführung des erfindungsgemäßen Verfahrens (a) innerhalb eines größeren Bereichs variiert werden. Im allgemeinen arbeitet man bei Temperaturen zwischen -20°C und +150°C, vorzugsweise zwischen 0°C und +120°C.

0095683

Das erfindungsgemäße Verfahren (a) wird im allgemeinen bei Normaldruck ausgeführt.

Bei der Durchführung des erfindungsgemäßen Verfahrens (a) werden die Ausgangsstoffe der Formeln (II) und (III) im allgemeinen in etwa äquimolaren Mengen eingesetzt. Ein Überschuß der einen oder anderen Komponente ist möglich. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung erfolgt nach üblichen Methoden.

Die neuen Verbindungen fallen zum Teil in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperatur von den letzten flüchtigen Anteilen befreit und auf diese Weise gereinigt werden können.

Zur Charakterisierung der neuen Substanzen dient der Brechungsindex. Soweit die neuen Produkte in fester Form anfallen, können sie durch Umkristallisation gereinigt werden. Zur Charakterisierung dient dann der Schmelzpunkt.

Die bei der Durchführung des erfindungsgemäßen Verfahrens (b) als Ausgangsstoffe benötigten Phenoxyphenole sind durch die Formel (IV) definiert. In dieser Formel haben $X^1$, $X^2$ und R vorzugsweise diejenigen Bedeutungen, die bereits im Zusammenhang mit der Beschreibung der erfindungsgemäßen Stoffe der Formel (I) vorzugsweise für diese Reste genannt wurden.

Z steht vorzugsweise für Wasserstoff, Natrium, Kalium oder für ein Calcium-Äquivalent.

Die Phenoxyphenole der Formel (IV) sind bekannt (vgl. US-PS 3 928 416).

Die außerdem bei dem Verfahren (b) als Ausgangsstoffe einzusetzenden Verbindungen sind durch die Formel (V) definiert. In dieser Formel haben $R^1$, $R^2$,$R^3$,$R^4$,$R^5$,$R^6$,Y,m, n und p vorzugsweise diejenigen Bedeutungen, die bereits im Zusammenhang mit der Beschreibung der erfindungsgemäßen Stoffe der Formel (I) vorzugsweise für diese Reste bzw. Indices genannt wurden. $Hal^1$ steht für Chlor oder Brom.

Die Verbindungen der Formel (V) sind neu. Sie lassen sich dadurch herstellen, daß man

α) Carbonylverbindungen der Formel

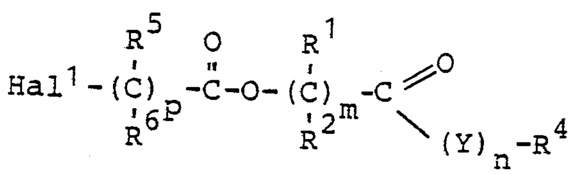

(XVIII)

in welcher
$R^1$, $R^2$, $R^3$, $R^4$, $R^5$, $R^6$, Y, $Hal^1$, m, n und p
die oben angegebene Bedeutung haben,

mit Hydroxylamin bzw. mit Hydroxylamin-Derivaten in Gegenwart eines Verdünnungsmittels, wie z.B. eines Alkohols, sowie gegebenenfalls in Gegenwart eines Säurebindemittels, wie z.B. Natriumacetat, bei Temperaturen zwischen 0°C und 100°C, vorzugsweise zwischen 0°C und 80°C umsetzt, wobei das Hydroxylamin bzw. das Hydroxylamin-Derivat vorzugsweise in Form eines Salzes, insbesondere als Hydrochlorid, eingesetzt wird,

oder

ß) Verbindungen der Formel

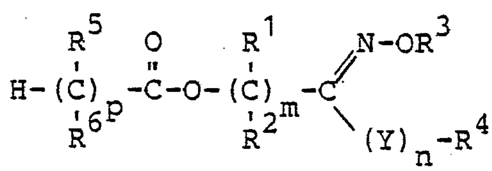

(XIX)

in welcher
$R^1$, $R^2$, $R^3$, $R^4$, $R^5$, $R^6$, Y, m, n und p die oben angegebene Bedeutung haben,
in üblicher Weise chloriert oder bromiert,

oder

γ) Alkohole der Formel

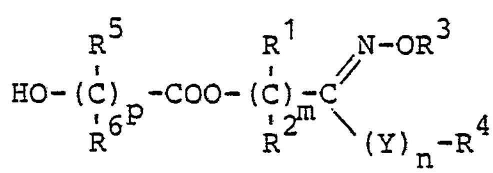

(XX)

in welcher
$R^1$, $R^2$, $R^3$, $R^4$, $R^5$, $R^6$, Y, m, n und p die oben angegebene Bedeutung haben,

mit Thionylchlorid oder Thionylbromid gegebenenfalls in Gegenwart eines Säureakzeptors und gegebenenfalls in Gegenwart eines Katalysators, wie z.B. Dimethylformamid, und gegebenenfalls in Gegenwart eines Verdünnungsmittels bei Temperaturen zwischen 0°C und 80°C umsetzt,

oder

δ) Alkohole der Formel

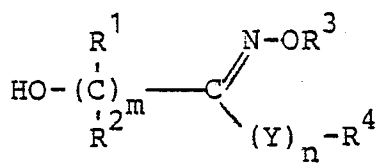

(XVI)

in welcher
$R^1$, $R^2$, $R^3$, $R^4$, Y, m und n die oben angegebene Bedeutung haben,

mit Carbonsäurehalogeniden der Formel

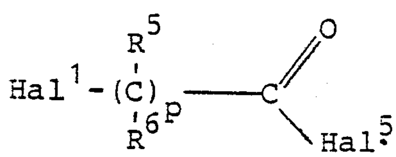

(XXI)

in welcher
$R^5$, $R^6$, $Hal^1$, $Hal^5$ und p die oben angegebene Bedeutung haben,

gegebenenfalls in Gegenwart eines Verdünnungsmittels, wie z.B. Tetrahydrofuran oder Toluol, sowie gegebenenfalls in Gegenwart eines Säureakzeptors, wie z.B. Pyridin oder Kaliumcarbonat, bei Temperaturen zwischen -20°C und +120°C, vorzugsweise zwischen 0°C und 80°C, umsetzt.

Als Säureakzeptoren können bei dem erfindungsgemäßen Verfahren (b) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (b) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen -20°C und +150°C, vorzugsweise zwischen 0°C und +120°C.

Das erfindungsgemäße Verfahren (b) wird im allgemeinen bei Normaldruck ausgeführt.

Bei der Durchführung des erfindungsgemäßen Verfahrens (b) werden die Ausgangsstoffe der Formeln (IV) und (V)

im allgemeinen in etwa äquimolaren Mengen eingesetzt. Ein Überschuß der einen oder anderen Komponente ist möglich. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung und die Reinigung der Substanzen erfolgen nach üblichen Methoden.

Die bei der Durchführung des erfindungsgemäßen Verfahrens (c) als Ausgangsstoffe benötigten Trifluormethyl-phenoxy-phenyl-Derivate sind durch die Formel (Ia) definiert. In dieser Formel haben $X^1$, $X^2$, X, R, $R^1$, $R^2$ und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die Verbindungen der Formel (Ia) sind neu; sie lassen sich jedoch nach den erfindungsgmäßen Verfahren (a), (b), (e), (g), (h), (i), (k) und (l) in einfacher Weise herstellen.

Die außerdem bei dem Verfahren (c) als Ausgangsstoffe einzusetzenden Dihalogenalkane sind durch die Formel (VI) definiert. In dieser Formel steht q für die Zahlen 1, 2 oder 3, und $Hal^2$ steht jeweils für Chlor oder Brom.

Die Verbindungen der Formel (VI) sind allgemein bekannte Verbindungen der organischen Chemie.

Als Säureakzeptoren können bei dem erfindungsgemäßen Verfahren (c) alle üblichen Säurebindemittel verwendet werden. Vorzugweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (c) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (c) innerhalb eines größeren Bereiches variiert werden. Im allgemeinen arbeitet man bei Temperaturen zwischen -20°C und +120°C, vorzugsweise zwischen 0°C und +80°C.

Das erfindungsgemäße Verfahren (c) wird im allgemeinen bei Normaldruck ausgeführt.

Bei der Durchführung des erfindungsgemäßen Verfahrens (c) werden die Ausgangsstoffe der Formeln (Ia) und (VI) im allgemeinen in etwa äquimolaren Mengen eingesetzt. Ein Überschuß der einen oder anderen Komponente ist möglich. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung erfolgt nach üblichen Methoden.

Sofern eine Reinigung der bei dem erfindungsgemäßen Verfahren (c) anfallenden Verbindungen erforderlich ist, so wird diese nach den Methoden vorgenommen, die bereits im Zusammenhang mit der Beschreibung des erfindungsgemäßen Verfahrens (a) genannt wurden.

Die bei der Durchführung des erfindungsgemäßen Verfahrens (d) als Ausgangsstoffe benötigten Trifluormethylphenoxy-phenyl-Derivate sind durch die Formel (Ib) definiert. In dieser Formel haben $X^1$, $X^2$, X, R, $R^1$, $R^2$, $R^4$, Y, m und n vorzugsweise diejenigen Bedeutungen, die bereits in Zusammenhang mit der Formel (I) vorzugsweise für diese Reste bzw. Indices genannt wurden.

Die Verbindungen der Formel (Ib) sind neu; sie lassen sich jedoch nach den erfindungsgemäßen Verfahren (a), (b), (e), (f), (g), (i), (k) und (l) in einfacher Weise herstellen.

Die weiterhin bei dem Verfahren (d) als Ausgangsstoffe einzusetzenden Alkylierungsmittel sind durch die Formel (VII) definiert. In dieser Formel steht $R^{3'}$ vorzugsweise für Methyl, Ethyl, n-Propyl und iso-Propyl. $Z^1$ steht für Chlor, Brom, eine p-Toluolsulfonyl-Gruppe oder für die Gruppierung $R^8O-SO_2-O-$, worin $R^8$ für Wasserstoff, Methyl, Ethyl, n-Propyl oder iso-Propyl steht.

Die Verbindungen der Formel (VII) sind allgemein bekannte Verbindungen der organischen Chemie.

Als Säureakzeptoren können bei dem erfindungsgemäßen Verfahren (d) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (d) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (d) innerhalb eines größeren Bereiches variiert werden. Im allgemeinen arbeitet man bei Temperaturen zwischen -20°C und +120°C, vorzugsweise zwischen -10°C und +100°C.

Das erfindungsgemäße Verfahren (d) wird im allgemeinen bei Normaldruck ausgeführt.

Bei der Durchführung des erfindungsgemäßen Verfahrens (d) werden die Ausgangsstoffe der Formeln (Ib) und (VII) im allgemeinen in etwa äquimolaren Mengen eingesetzt. Ein Überschuß der einen oder anderen Komponente ist möglich. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung und die Reinigung der Substanzen erfolgen nach üblichen Methoden.

Die bei der Durchführung des erfindungsgemäßen Verfahrens (e) als Ausgangsstoffe benötigten Halogenide sind durch die Formel (VIII) definiert. In dieser Formel haben $X^1$, $X^2$, X, R, $R^1$, $R^2$, $R^3$ und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden. $Hal^3$ steht für Chlor oder Brom.

Die Halogenide der Formel (VIII) sind neu. Sie lassen sich herstellen, indem man Hydroxamsäure-Derivate der Formel

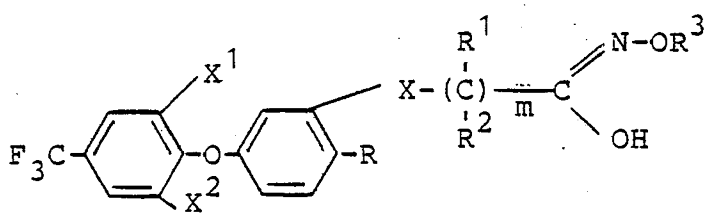

(Ih)

in welcher
$X^1$, $X^2$, X, R, $R^1$, $R^2$, $R^3$ und m die oben angegebene Bedeutung haben, bzw. deren tautomere Verbindungen oder Aldoxime der Formel

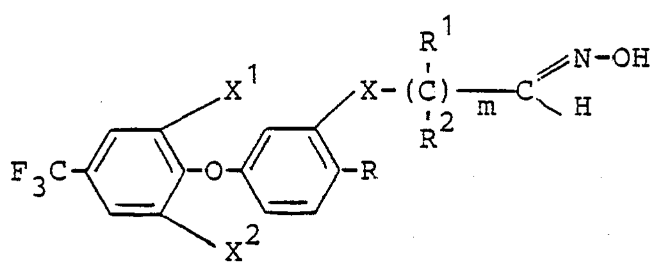

(Ii)

in welcher
$X^1$, $X^2$, X, R, $R^1$, $R^2$ und m die oben angegebene Bedeutung haben,

mit Chlorierungs- oder Bromierungsmitteln, wie zum Beispiel Phosphorpentachlorid, Phosphortribromid, Chlor oder Brom, gegebenenfalls in Gegenwart eines Säureakzeptors, wie zum Beispiel Pyridin, bei Temperaturen zwischen -40°C und +60°C umsetzt (vgl. Houben-Weyl, "Methoden der organischen Chemie", Band VIII, Seiten 691-692).

Die Verbindungen der Formeln (Ih) und (Ii) sind ebenfalls neu; sie lassen sich jedoch nach den erfindungsgemäßen Verfahren (a) - (i) in einfacher Weise herstellen.

Die weiterhin bei dem Verfahren (e) als Ausgangsstoffe einzusetzenden Verbindungen sind durch die Formel (IX) definiert. In dieser Formel hat $R^4$ vorzugsweise diejenigen Bedeutungen, die bereits im Zusammenhang mit der Beschreibung der erfindungsgemäßen Stoffe der Formel (I) vorzugsweise diesen Rest genannt wurden. $Z^2$ steht vorzugsweise für Wasserstoff oder ein Äquivalent eines Natrium-, Kalium- oder Calciumions. Q steht vorzugsweise für Sauerstoff, Schwefel oder für $NR^7$, worin $R^7$ vorzugsweise für Wasserstoff, Methyl, Ethyl, n-Propyl oder iso-Propyl steht. - Die Verbindungen der Formel (IX) sind bekannt.

Als Säureakzeptoren können bei dem erfindungsgemäßen Verfahren (e) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des

erfindungsgemäßen Verfahrens (e) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (e) innerhalb eines größeren Bereiches variiert werden. Im allgemeinen arbeitet man bei Temperaturen zwischen -20 und +150°C, vorzugsweise zwischen -10 und +120°C.

Das erfindungsgemäße Verfahren (e) wird im allgemeinen bei Normaldruck ausgeführt.

Bei der Durchführung des erfindungsgemäßen Verfahrens (e) werden die Ausgangsstoffe der Formeln (VIII) und (IX) im allgemeinen in etwa äquimolaren Mengen eingesetzt. Ein Überschuß der einen oder anderen Komponente ist möglich. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung und die Reinigung der Substanzen erfolgen nach üblichen Methoden.

Die bei der Durchführung des erfindungsgemäßen Verfahrens (f) als Ausgangsstoffe benötigten Nitrile sind durch die Formel (X) definiert. In dieser Formel haben $X^1$, $X^2$, X, R, $R^1$, $R^2$ und m vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden.

Die Verbindungen der Formel (X) sind teilweise bekannt (vgl. DE-OS 2 613 697). Sie können erhalten werden, indem man Verbindungen der Formel (II) mit entsprechenden Halogen-nitrilen in Gegenwart eines inerten organischen Lösungsmittels, wie z.B. Acetonitril und gegebenenfalls in Gegenwart eines Säurebindemittels, wie z.B. Kaliumcarbonat, sowie gegebenenfalls in Gegenwart eines Katalysators, wie z.B. Natriumiodid, bei Temperaturen zwischen 0 und 100°C umsetzt.

Als Säureakzeptoren können bei dem erfindungsgemäßen Verfahren (f) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (f) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (f) innerhalb eines größeren Bereichs variiert werden. Im allgemeinen arbeitet man bei Temperaturen zwischen -20 und +120°C, vorzugsweise zwischen -10 und +100°C.

Das erfindungsgemäße Verfahren (f) wird im allgemeinen bei Normaldruck ausgeführt.

Bei der Durchführung des erfindungsgemäßen Verfahrens (f) setzt man vorzugsweise auf 1 Mol der Verbindungen der Formel (X), 1 bis 2 Mol Hydroxylamin bzw. das entsprechende Hydrochlorid oder Hydrobromid ein. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung und die Reinigung der Substanzen erfolgen nach üblichen Methoden.

Die bei der Durchführung des erfindungsgemäßen Verfahrens (g) als Ausgangsstoffe benötigten Verbindungen sind durch die Formel (XI) definiert. In dieser Formel haben $X^1$, $X^2$, X, R, $R^1$, $R^2$, $R^4$ und m vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden. $Y^1$ steht für Sauerstoff oder Schwefel.

Die Verbindungen der Formel (XI) sind neu. Sie lassen sich herstellen, indem man Nitrile der Formel

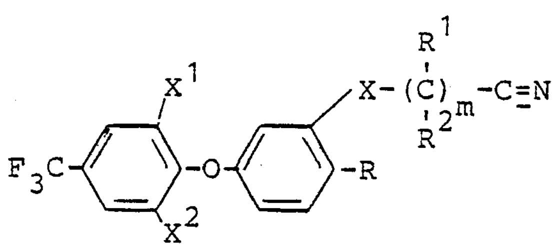

(X)

in welcher

$X^1$, $X^2$, X, $R^1$, $R^2$, R und m die oben angegebene Bedeutung haben,

mit Verbindungen der Formel

$$R^4 - Y^1 - H \qquad \text{(XXII)}$$

in welcher $R^4$ und $Y^1$ die oben angegebene Bedeutung haben,

in Gegenwart von Chlorwasserstoff und in Gegenwart eines Lösungsmittels, wie beispielsweise Toluol, bei Temperaturen zwischen -10°C und 30°C umsetzt (vergl. DE-OS 2 746 057 und Ber. Dtsch. Chem. Ges. 16, 356 (1883)).

Die Verbindungen der Formel (XXII) sind allgemein bekannte Verbindungen der organischen Chemie.

Als Säureakzeptoren können bei dem erfindungsgemäßen Verfahren (g) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (g) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (g) innerhalb eines

größeren Bereiches variiert werden. Im allgemeinen arbeitet man bei Temperaturen zwischen -20 und +50°C, vorzugsweise zwischen -10 und +30°C.

Das erfindungsgemäße Verfahren (g) wird im allgemeinen bei Normaldruck ausgeführt.

Bei der Durchführung des erfindungsgemäßen Verfahrens (g) setzt man vorzugsweise auf 1 Mol der Verbindungen der Formel (XI), 1 bis 2 Mol Hydroxylamin bzw. Hydroxylaminhydrochlorid oder Hydroxylaminhydrobromid ein. Die Umsetzung wird im allgemeinen in einem geeigneten Verdünnungsmittel in Gegenwart eines Säureakzeptors durchgeführt, und das Reaktionsgemisch wird bei der erforderlichen Temperatur gerührt. Die Aufarbeitung und die Reinigung der Substanzen erfolgen nach üblichen Methoden.

Die bei der Durchführung des erfindungsgemäßen Verfahrens (h) als Ausgangsstoffe benötigten Trifluormethyl-phenoxy-phenyl-Derivate, sind durch die Formel (Ic) definiert. In dieser Formel haben X, $X^1$, $X^2$, $R^1$, $R^2$, $R^3$, $R^4$, Y, m und n vorzugsweise diejenigen Bedeutungen, die bereits im Zusammenhang mit der Beschreibung der erfindungsgemäßen Stoffe der Formel (I) vorzugsweise für diese Reste bzw. Indices genannt wurden.

Die bei dem erfindungsgemäßen Verfahren (h) als Ausgangsstoffe benötigten Verbindungen der Formel (Ic) sind neu; sie lassen sich jedoch nach den erfindungsgemäßen Verfahren (a) - (g) und Verfahren (i), (k) und (l) in einfacher Weise herstellen.

Zur Nitrierung verwendet man im Falle des erfindungsgemäßen Verfahrens (h) vorzugsweise konzentrierte Salpetersäure.

Das erfindungsgemäße Verfahren (h) wird gegebenenfalls unter Verwendung eines Verdünnungsmittels durchgeführt. Als Verdünnungsmittel kommen hierbei vorzugsweise halogenierte Kohlenwasserstoffe, wie z.B. Methylenchlorid, in Betracht.

Das erfindungsgemäße Verfahren (h) wird gegebenenfalls in Gegenwart eines Katalysators durchgeführt. Als Katalysatoren kommen vorzugsweise Protonensäuren, wie z.B. Schwefelsäure oder Essigsäure, in Betracht.

Die Reaktionstemperatur wird bei Verfahren (h) im allgemeinen zwischen -20 und +80°C, vorzugsweise zwischen 0 und 50°C gehalten. Verfahren (h) wird im allgemeinen bei Normaldruck durchgeführt.

Zur Durchführung des erfindungsgemäßen Verfahrens (h) setzt man auf 1 Mol einer Verbindung mit der Formel (Ic) im allgemeinen 0,9 bis 2 Mol, vorzugsweise 1,0 bis 1,3 Mol an Salpetersäure und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt.

Die Aufarbeitung erfolgt nach üblichen Methoden. Im allgemeinen verfährt man in der Weise, daß man das Reaktionsgemisch nach beendeter Umsetzung in Eiswasser eingießt, absaugt und das anfallende Produkt gegebenenfalls durch Umkristallisieren reinigt.

Die beim erfindungsgemäßen Verfahren (i) als Ausgangsstoffe benötigten Carbonyl-Derivate sind durch die Formel (XII) definiert. In dieser Formel haben X, $X^1$, $X^2$, R, $R^1$, $R^2$ und m vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden. $R^9$ steht vorzugsweise für Wasserstoff oder $C_1$-$C_4$-Alkyl.

Die Carbonyl-Derivate der Formel (XII) sind bekannt oder lassen sich nach üblichen Methoden in einfacher Weise herstellen (vgl. DE-AS 2 304 006, GB-PS 2 058 055 und EP-OS 28 277).

Als Säureakzeptoren können bei dem erfindungsgemäßen Verfahren (i) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (i) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (i) innerhalb eines größeren Bereiches variiert werden. Im allgemeinen arbeitet man bei Temperaturen zwischen -20 und +120°C, vorzugsweise zwischen -10 und +80°C.

Das erfindungsgemäße Verfahren (i) wird im allgemeinen bei Normaldruck ausgeführt.

Bei der Durchführung des erfindungsgemäßen Verfahrens (i) setzt man vorzugsweise auf 1 Mol Carbonyl-Derivat der Formel (XII) 1 bis 2 Mol Hydroxylamin oder Hydroxylaminhydrochlorid ein. Die Umsetzung wird im allgemeinen in einem geeigneten Verdünnungsmittel in Gegenwart eines Säureakzeptors durchgeführt, und das Reaktionsgemisch wird bei der erforderlichen Temperatur gerührt. Die Aufarbeitung und die Reinigung der Substanzen erfolgt nach üblichen Methoden.

Die beim erfindungsgemäßen Verfahren (k) benötigten Trifluormethylbenzole sind durch die Formel (XIII) definiert. In dieser Formel haben $X^1$ und $X^2$ vorzugsweise diejenigen Bedeutungen, die bereits im Zusammenhang mit der Beschreibung der erfindungsgemäßen Stoffe der Formel (I) vorzugsweise für diese Reste genannt wurden. $Hal^4$ steht für Chlor und Brom.

Die Trifluormethylbenzole der Formel (XIII) sind allgemein bekannte Verbindungen der organischen Chemie.

Die weiterhin für das Verfahren (k) als Ausgangsstoffe

benötigten Verbindungen sind durch Formel (XIV) definiert. In dieser Formel haben X, R, $R^1$, $R^2$, $R^3$, $R^4$, Y, m und n vorzugsweise diejenigen Bedeutungen, die bereits im Zusammenhang mit der Beschreibung der erfindungsgemäßen Stoffe der Formel (I) vorzugsweise für diese Reste bzw. Indices genannt wurden und $Z^3$ steht vorzugsweise für Wasserstoff, Natrium oder Kalium oder für ein Äquivalent eines Calciumions.

Die Verbindungen der Formel (XIV) sind teilweise bekannt. Sie lassen sich analog den erfindungsgemäßen Verfahren (a), (b), (c), (d), (e), (f), (g) und (i) in einfacher Weise herstellen.

Als Säureakzeptoren können bei dem erfindungsgemäßen Verfahren (k) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (k) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (k) innerhalb eines größeren Bereiches variiert werden. Im allgemeinen

arbeitet man bei Temperaturen zwischen 0°C und 180°C, vorzugsweise zwischen 60° und 140°C.

Das erfindungsgemäße Verfahren (k) wird im allgemeinen bei Normaldruck ausgeführt.

Bei der Durchführung des erfindungsgemäßen Verfahrens (k) werden die Ausgangsstoffe der Formeln (XIII) und (XIV) im allgemeinen in etwa äquimolaren Mengen eingesetzt. Ein Überschuß der einen oder anderen Komponente ist möglich. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung und die Reinigung der Substanzen erfolgen nach üblichen Methoden.

Die für das erfindungsgemäße Verfahren (l) als Ausgangsstoffe benötigten Säurehalogenide sind durch die Formel (XV) definiert. In dieser Formel haben $X^1$, $X^2$ und R vorzugsweise diejenigen Bedeutungen, die bereits im Zusammenhang mit der Beschreibung der erfindunsgemäßen Stoffe der Formel (I) vorzugsweise für diese Reste genannt wurden, und A steht für eine -CO-Gruppe oder die Gruppierung der Formel

$$-O-(\overset{R^5}{\underset{R^6}{C}})_p-CO-$$

in welcher $R^5$, $R^6$ und p vorzugsweise diejenigen Bedeutungen haben, die bereits im Zusammenhang mit der Beschreibung der erfindungsgemäßen Verbindungen der Formel (I) vorzugsweise für diese Reste bzw. für diesen Index genannt wurden. $Hal^5$ steht für Chlor oder Brom.

Die Verbindungen der Formel (XV) sind bekannt oder lassen sich nach bekannten Verfahren in einfacher Weise herstellen (vgl. DE-OS 2 906 087).

Die weiterhin für das Verfahren (l) als Ausgangsstoffe benötigten Verbindungen sind durch die Formel (XVI) definiert. In dieser Formel haben $R^1$, $R^2$, $R^3$, $R^4$, Y, m und n vorzugsweise diejenigen Bedeutungen, die bereits im Zusammenhang mit der Beschreibung der erfindungsgemäßen Stoffe der Formel (I) vorzugsweise für diese Reste bzw. Indices genannt wurden.

Die Verbindungen der Formel (XVI) sind bereits bekannt (vgl. DE-OS 31 00 728).

Als Säureakzeptoren können bei dem erfindungsgemäßen Verfahren (l) alle üblichen Säurebindemittel verwendet werden. Vorzugweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (l) praktisch alle inerten organischen Solventien verwendet werden. Vorzugsweise in Betracht kommen diejenigen Verdünnungsmittel, die im

Zusammenhang mit der Beschreibung des erfindungsgemäßen Verfahrens (a) genannt wurden.

Die Reaktionstemperaturen können bei der Durchführung des erfindungsgemäßen Verfahrens (1) innerhalb eines größeren Bereiches variiert werden. Im allgemeinen arbeitet man bei Temperaturen zwischen -40 und +160°C, vorzugsweise zwischen 0 und +110°C.

Das erfindungsgemäße Verfahren (1) wird im allgemeinen bei Normaldruck ausgeführt.

Bei der Durchführung des erfindungsgemäßen Verfahrens (1) werden die Ausgangsstoffe der Formeln (XV) und (XVI) im allgemeinen in etwa äquimolaren Mengen eingesetzt. Ein Überschuß der einen oder anderen Komponente ist möglich. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die Aufarbeitung und die Reinigung der Substanzen erfolgen nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale und selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe sind besonders gut zur selektiven Unkrautbekämpfung in verschiedenen wichtigen Kulturen, wie zum Beispiel Rüben und Sojabohnen, geeignet. Außerdem lassen sich die erfindungsgemäßen Wirkstoffe sehr gut zur Entlaubung und zur Austrocknung der Blätter bei Baumwolle verwenden. Weiterhin besitzen die erfindungsgemäßen Wirkstoffe auch eine insektizide Wirksamkeit.

Die Wirkstoffe können in die üblichen Formulierungen überführt werden, wie Lösungen, Emulsionen, Spritzpulver, Suspensionen, Pulver, Stäubemittel, Pasten, Granulate, Suspensions-Emulsions-Konzentrate, wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Gylcol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden oder Pflanzenwachstumsregulatoren zur Unkrautbekämpfung bzw. als Pflanzenwuchsregulatoren Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4 (1H, 3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5 (4H)-on zur

Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei einer Anwendung als Herbizide sowohl vor als auch nach dem Auflaufen der Pflanzen appliziert werden. Die Anwendung wird vorzugsweise nach dem Auflaufen der Pflanzen, also im post-emergence-Verfahren, vorgenommen. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 50 kg Wirkstoff pro

Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

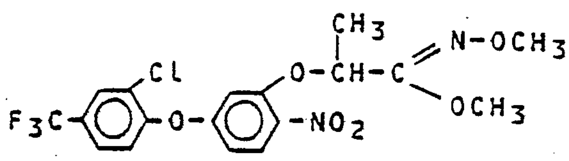

(Verfahren a)

In eine Lösung von 22 g (0,066 Mol) 5-(2-Chlor-4-trifluormethylphenoxy)-2-nitro-phenol und 11 g (0,08 Mol) Kaliumcarbonat in 100 ml Acetonitril werden bei 50-60°C 15 g (0,076 Mol) 2-Brom-1-methoximino-1-methoxy-propan eingetropft. Anschließend wird 25 Stunden unter Rückfluß erhitzt. Das Reaktionsgemisch wird eingeengt, und der Rückstand wird in Wasser aufgenommen. Die sich abscheidenden Kristalle werden abgesaugt. Man erhält 27,6 g (96 % der Theorie) 2-/5-(2-Chlor-4-trifluormethylphenoxy)-2-nitro-phenoxy/-1-methoxyimino-1-methoxypropan vom Schmelzpunkt 90-91°C.

Vorprodukt:

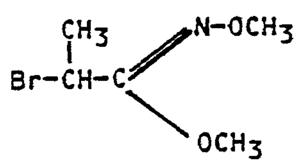

(III-1)

8,2 g (0,07 Mol) Propionhydroximsäure-0,0-dimethylester und 12,46 g (0,07 Mol) N-Brom-succinimid werden in 100 mL Tetrachlorkohlenstoff unter UV-Bestrahlung mit einer 300 Watt-Lampe 4 Stunden unter Rückfluß gekocht. Anschließend wird das abgeschiedene Succinimid abfiltriert, und das Filtrat wird im Wasserstrahlvakuum destilliert. Man erhält 7,6 g (55 % der Theorie) 2-Brom-1-methoximino-1-methoxy-propan vom Siedepunkt 65-75°C/12 mm Hg.

Beispiel 2

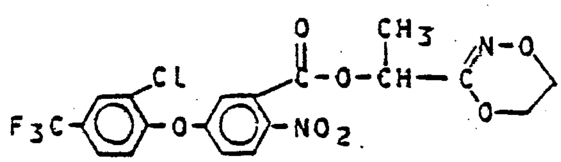

(Verfahren a)

10,9 g (0,03 Mol) 5-(2-Chlor-4-trifluormethyl-phenoxy)-2-nitrobenzoesäure in 80 ml Aceton werden mit 9 g 1,8-Diazabicyclo/5,4,0/undecen-7 versetzt. Anschließend werden bei 50-55°C 5,9 g (0,03 Mol) 3-(α-Bromethyl)-1,4-diox-2-azin zugetropft, und es wird 8 Stunden nachgerührt. Anschließend wird das Lösungsmittel abgezogen und der Rückstand in Toluol aufgenommen. Die organische Phase wird nacheinander mit verdünnter wäßriger Natronlauge, verdünnter Salzsäure und Wasser gewaschen. Danach wird das Lösungsmittel abdestilliert. Man erhält 13,3 g (93,5 % der Theorie) 5-(2-Chlor-4-trifluormethyl-phenoxy)-2-nitro-

benzoesäure-1-(1,4-diox-2-azin-3-yl)-ethylester vom Brechnungsindex $n_D^{20}$ = 1,5437.

Vorprodukte

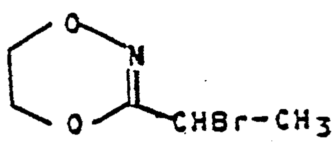

(III-2)

34,5 g (0,3 Mol) 3-Ethyl-1,4-diox-2-azin und 54 g (0,3 Mol) N-Bromsuccinimid werden in 300 ml Tetrachlorkohlenstoff bei UV-Belichtung 75 Minuten unter Rückfluß erhitzt. Nach Filtration und Einengen im Vakuum wird der Rückstand destilliert. Man erhält 44,8 g (77 % der Theorie) 3-( α-Bromethyl)-1,4-diox-2-azin vom Brechungsindex $n_D^{20}$ = 1,5086.

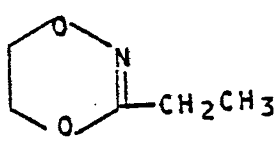

(XVII-1)

105 g (1,5 Mol) Hydroxylamin-hydrochlorid, gelöst in 660 ml Methanol werden bei 40°C mit 168 g (3 Mol) Kaliumhydroxid, in 650 ml Methanol gelöst, versetzt. Dann gibt man 132 g (1,5 Mol) Propionsäuremethylester zu, läßt 16 Stunden stehen und filtriert danach das ausgefallene Kaliumchlorid ab. Die Mutterlauge wird mit 207 g (1,5 Mol) Kaliumcarbonat unter Rühren auf 40°C erwärmt und nach

tropfenweiser Zugabe von 282 g (1,5 Mol) 1,2-Dibromethan 30 Stunden bei gleicher Temperatur gehalten. Danach filtriert man, extrahiert die Salzphase noch mehrfach mit Ether und engt die organischen Anteile ein. Nach Destillation erhält man 65,1 g (38 % der Theorie) 3-Ethyl-1,4-diox-2-azin vom Brechungsindex $n_D^{20}$ = 1,452.

Nach der in den vorausgehenden Beispielen beschriebenen Methode oder auch nach anderen erfindungsgemäßen Verfahren werden die in der folgenden Tabelle 3 formelmäßig aufgeführten Verbindungen hergestellt.

Tabelle 3

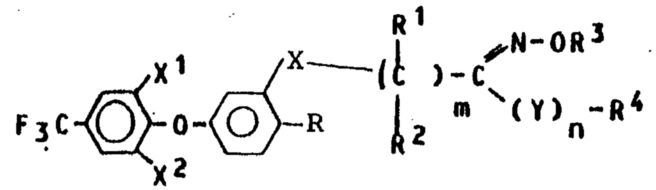

(I)

| Beispiel Nr. | X | $X^1$ | $X^2$ | R | $R^1$ | $R^2$ | $R^3$ | $R^4$ | m | Y | n | Schmelzpunkt [°C] bzw. Brechungsindex [$n_D^{20}$] |
|---|---|---|---|---|---|---|---|---|---|---|---|---|
| 3 | O | Cl | H | $NO_2$ | $-CH_3$ | H | $-CH_2-CH_2-$ | | 1 | O | 1 | 135 |
| 4 | O | Cl | H | $NO_2$ | $-CH_3$ | H | $-C_2H_5$ | $-C_2H_5$ | 1 | O | 1 | 176 |
| 5 | O | Cl | H | $NO_2$ | $-CH_3$ | H | $-CH_3$ | $-C_2H_5$ | 1 | O | 1 | 1,5313 |
| 6 | O | Cl | H | $NO_2$ | $-CH_3$ | H | $-CH_3$ | $-CH_3$ | 1 | S | 1 | 1,5576 |
| 7 | O | Cl | Cl | $NO_2$ | $-CH_3$ | H | $-CH_2-CH_2-$ | | 1 | O | 1 | 140 |
| 8 | O | Cl | Cl | $NO_2$ | $-CH_3$ | H | $-CH_3$ | $-CH_3$ | 1 | S | 1 | 87 |
| 9 | O | Cl | Cl | $NO_2$ | $-CH_3$ | H | $-CH_3$ | H | 1 | - | o | 95 |
| 10 | O | Cl | Cl | $NO_2$ | $-CH_3$ | H | $-C_2H_5$ | H | 1 | - | o | 84 |
| 11 | O | Cl | H | $NO_2$ | $-CH_3$ | H | $-CH_3$ | H | 1 | - | o | 1,5463 |
| 12 | O | Cl | H | $NO_2$ | $-CH_3$ | H | $-C_2H_5$ | H | 1 | - | o | 1,5405 |
| 13 | O | Cl | Cl | $NO_2$ | H | H | $-CH_3$ | H | 1 | - | o | 71 |
| 14 | O | Cl | Cl | $NO_2$ | H | $-CH_2-CH_2-$ | | $-C_2H_5$ | 1 | O | 1 | 118 |

Tabelle 3 (Fortsetzung)

| Beispiel Nr. | X | $X^1$ | $X^2$ | R | $R^1$ | $R^2$ | $R^3$ | $R^4$ | m | Y | n | Schmelzpunkt [°C] bzw. Brechungsindex [$n^{20}_D$] |
|---|---|---|---|---|---|---|---|---|---|---|---|---|
| 15 | O | Cl | H | $NO_2$ | H | $-CH_2-CH_2$ | | $-C_2H_5$ | 1 | O | 1 | 105 |
| 16 | O | $NO_2$ | H | H | $-CH_3$ | H | $-CH_2-CH_2-$ | | 1 | O | 1 | 110-116 |
| 17 | O | $NO_2$ | H | H | $-CH_3$ | H | $-CH_3$ | $-CH_3$ | 1 | O | 1 | 1,5215 |
| 18 | O | $NO_2$ | H | H | $-CH_3$ | H | $-C_2H_5$ | $-CH_3$ | 1 | S | 1 | 1,5418 |
| 19 | O | Cl | Cl | H | $-CH_3$ | H | $-CH_3$ | $-C_2H_5$ | 1 | $CO_2$ | 1 | 1,5149 |
| 20 | O | Cl | Cl | H | $-CH_3$ | H | $-CH_3$ | $-CH_3$ | 1 | S | 1 | 1,5490 |
| 21 | O | Cl | Cl | H | H | $-CH_2-CH_2-$ | | $-C_2H_5$ | 1 | O | 1 | 1,5291 |
| 22 | O | Cl | Cl | H | H | $-CH_3$ | H | H | 1 | -NH | 1 | 96 |
| 23 | $CO_2$ | Cl | H | $NO_2$ | $-CH_3$ | H | $-C_2H_5$ | H | 1 | - | o | 1,5348 |
| 24 | $CO_2$ | Cl | H | $NO_2$ | $-CH_3$ | H | $-CH_3$ | H | 1 | - | o | 1,5415 |

Tabelle 3 (Fortsetzung)

| Beispiel Nr. | X | $X^1$ | $X^2$ | R | $R^1$ | $R^2$ | $R^3$ | $R^4$ | m | Y | n | Schmelzpunkt [°C] bzw. Brechungsindex [$n_D^{20}$] |
|---|---|---|---|---|---|---|---|---|---|---|---|---|
| 25 | $-O-CH(CH_3)-CO_2-$ | Cl | Cl | H | $-CH_3$ | H | $-CH_2-CH_2-$ | | 1 | O | 1 | 1,4921 |
| 26 | $-O-CH(CH_3)-CO_2-$ | Cl | Cl | H | $-CH_3$ | H | $-CH_3$ | $-C_2H_5$ | 1 | O | 1 | 1,5130 |
| 27 | $-O-CH(CH_3)-CO_2-$ | Cl | Cl | H | $-CH_3$ | H | $-C_2H_5$ | H | 1 | O | 1 | 1,5132 |
| 28 | $CO_2$ | Cl | H | $NO_2$ | H | H | $-CH_3$ | H | 1 | - | O | 1,5510 |
| 29 | $CO_2$ | Cl | Cl | $NO_2$ | H | H | $-CH_3$ | H | 1 | - | O | $n_D^{22}$ = 1,5583 |
| 30 | $CO_2$ | Cl | Cl | $NO_2$ | $CH_3$ | H | $-CH_3$ | H | 1 | - | O | $n_D^{21,5}$ = 1,5395 |

In den folgenden Verwendungsbeispielen wird die nachstehend angegebene Verbindung als Vergleichssubstanz eingesetzt:

(A)

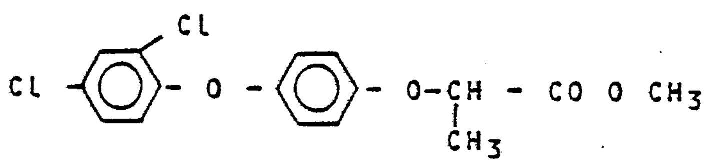

2-/4-(2,4-Dichlorphenoxy)-phenoxy7-propionsäure-methylester (bekannt aus DE-OS 2 223 894).

Beispiel A

Post-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe eine bessere selektive herbizide Wirksamkeit als die Vergleichssubstanz (A).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen die erfindungsgemäßen Wirkstoffe eine sehr gute herbizide Wirksamkeit.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall

In diesem Test zeigen die erfindungsgemäßen Wirkstoffe (1), (2), (5), (6), (9) und (13) eine starke bis sehr starke Wirksamkeit.

Patentansprüche

1. Trifluormethylphenoxy-phenyl-Derivate der Formel

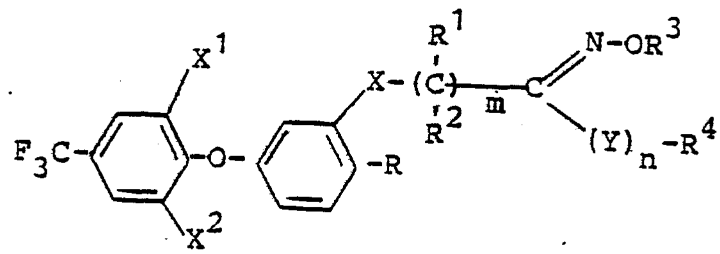

(I)

in welcher

$X^1$ für Wasserstoff, Fluor, Chlor, Brom, Jod, Nitro oder Cyano steht,

$X^2$ für Wasserstoff, Fluor, Chlor, Brom oder Jod steht,

R für Wasserstoff oder Nitro steht,

X für Sauerstoff, eine CO-O-Gruppe oder für die Gruppierung der Formel

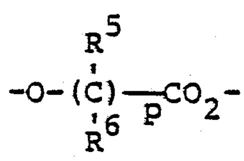

steht,

$R^1$, $R^2$, $R^5$ und $R^6$ unabhängig voneinander für Wasserstoff oder $C_1$-$C_5$-Alkyl stehen,

$R^3$ für Wasserstoff oder $C_1$-$C_5$-Alkyl steht, oder

$R^2$ und $R^3$ gemeinsam für $C_1$-$C_3$-Alkandiyl stehen,

$R^4$ für Wasserstoff oder $C_1$-$C_5$-Alkyl steht, oder

$R^3$ und $R^4$ gemeinsam für $C_1$-$C_3$-Alkandiyl stehen,

Y für Sauerstoff, Schwefel, eine $-NR^7$-Gruppe oder eine CO-O-Gruppe steht,

$R^7$ für Wasserstoff oder $C_1$-$C_5$-Alkyl steht,

m für die Zahlen 1, 2 oder 3 steht,

n für die Zahlen 0 oder 1 steht und

p für die Zahlen 1, 2 oder 3 steht.

2. Trifluormethylphenoxy-phenyl-Derivate der Formel (I), in denen

$X^1$ für Wasserstoff, Fluor, Chlor, Nitro oder Cyano steht,

$X^2$ für Wasserstoff, Fluor oder Chlor steht,

R für Wasserstoff oder Nitro steht,

X für Sauerstoff, eine -COO-Gruppe oder für die Gruppierung der Formel

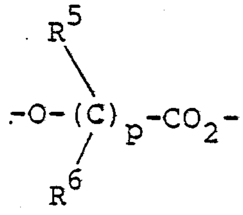

steht,

$R^1$, $R^2$, $R^5$ und $R^6$ unabhängig voneinander für Wasserstoff oder Methyl stehen,

$R^3$ für Wasserstoff, Methyl, Ethyl, n-Propyl oder Iso-Propyl steht,

oder

$R^2$ und $R^3$ gemeinsam für Ethandiyl stehen,

$R^4$ für Wasserstoff, Methyl, Ethyl, n-Propyl oder iso-Propyl steht,

oder

$R^3$ und $R^4$ gemeinsam für Ethandiyl stehen,

Y für Sauerstoff, Schwefel, eine $-NR^7-$Gruppe oder eine -CO-O-Gruppe steht,

$R^7$ für Wasserstoff, Methyl, Ethyl, n-Propyl oder iso-Propyl steht,

m für die Zahl 1 steht,

n für die Zahlen 0 oder 1 steht und

p für die Zahl 1 steht.

3. R- bzw. S-Enantiomere von Verbindungen der Formel

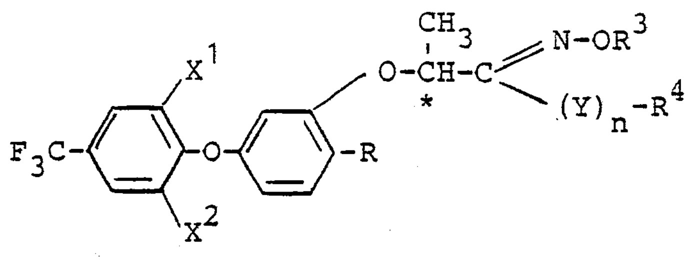

(Id)

in welcher

$X^1$ für Wasserstoff, Fluor, Chlor, Nitro oder Cyano steht,

$X^2$ für Wasserstoff, Fluor oder Chlor steht,

R für Wasserstoff oder Nitro steht,

$R^3$ für Wasserstoff, Methyl, Ethyl, n-Propyl oder iso-Propyl steht,

$R^4$ für Wasserstoff, Methyl, Ethyl, n-Propyl oder iso-Propyl steht, oder

$R^3$ und $R^4$ gemeinsam für Ethandiyl stehen,

Y für Sauerstoff, Schwefel, eine $-NR^7$-Gruppe oder eine -COO-Gruppe steht,

$R^7$ für Wasserstoff, Methyl, Ethyl, n-Propyl oder iso-Propyl steht und

n für die Zahlen 0 oder 1 steht.

4. R- bzw. S-Enantiomere von Verbindungen der Formel

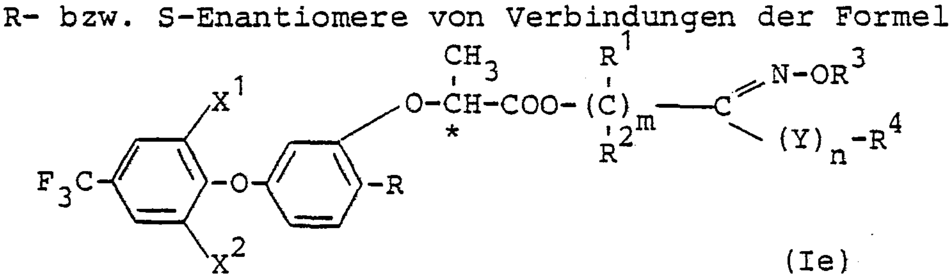

(Ie)

in welcher

$X^1$ für Wasserstoff, Fluor, Chlor, Nitro oder Cyano steht,

$X^2$ für Wasserstoff, Fluor oder Chlor steht,

R für Wasserstoff oder Nitro steht,

$R^1$ und $R^2$ unabhängig voneinander für Wasserstoff oder Methyl stehen,

$R^3$ für Wasserstoff, Methyl, Ethyl, n-Propyl oder iso-Propyl steht, oder

$R^2$ und $R^3$ gemeinsam für Ethandiyl stehen,

$R^4$ für Wasserstoff, Methyl, Ethyl, n-Propyl oder iso-Propyl steht, oder

$R^3$ und $R^4$ gemeinsam für Ethandiyl stehen,

Y für Sauerstoff, Schwefel, eine $-NR^7$-Gruppe oder eine -COO-Gruppe steht,

$R^7$ für Wasserstoff, Methyl, Ethyl, n-Propyl oder iso-Propyl steht,

m für die Zahl 1 steht und

n für die Zahlen 0 oder 1 steht.

5. Verfahren zur Herstellung von Trifluormethyl-phenoxyphenyl-Derivaten der Formel

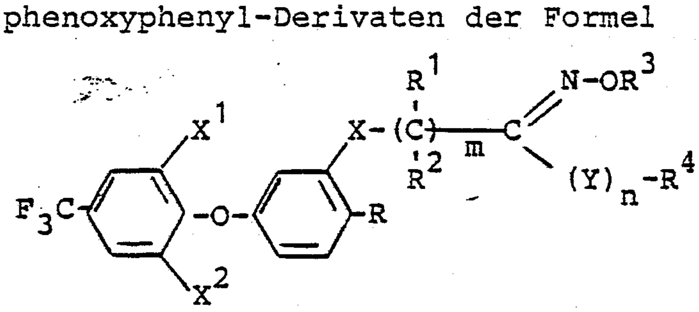

(I)

in welcher

$X^1$ für Wasserstoff, Fluor, Chlor, Brom, Jod, Nitro oder Cyano steht,

$X^2$ für Wasserstoff, Fluor, Chlor, Brom oder Jod steht,

R für Wasserstoff oder Nitro steht,

X für Sauerstoff, eine CO-O-Gruppe oder für die Gruppierung der Formel

$$-O-(\overset{R^5}{\underset{R^6}{C}})_p-CO_2-$$

steht,

$R^1$, $R^2$, $R^5$ und $R^6$ unabhängig voneinander für Wasserstoff oder $C_1$-$C_5$-Alkyl stehen,

$R^3$ für Wasserstoff oder $C_1$-$C_5$-Alkyl steht,

oder

$R^2$ und $R^3$ gemeinsam für $C_1$-$C_3$-Alkandiyl stehen,

$R^4$ für Wasserstoff oder $C_1$-$C_5$-Alkyl steht,

oder

$R^3$ und $R^4$ gemeinsam für $C_1$-$C_3$-Alkandiyl stehen,

Y für Sauerstoff, Schwefel, eine $-NR^7$-Gruppe oder eine CO-O-Gruppe steht,

$R^7$ für Wasserstoff oder $C_1$-$C_5$-Alkyl steht,

m für die Zahlen 1, 2 oder 3 steht,

n für die Zahlen 0 oder 1 steht und

p für die Zahlen 1, 2 oder 3 steht,

dadurch gekennzeichnet, daß man

a) Diphenylether der Formel

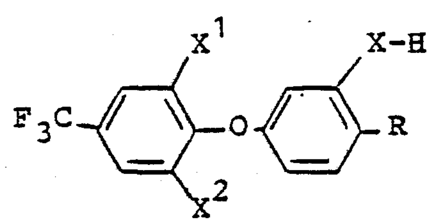

(II)

in welcher

$X^1$, $X^2$, X und R die oben angegebene Bedeutung haben,

mit Verbindungen der Formel

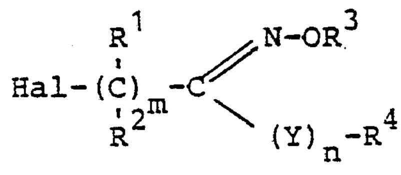

(III)

in welcher

$R^1$, $R^2$, $R^3$, $R^4$, Y, m und n die oben angegebene Bedeutung haben und

Hal für Chlor oder Brom steht,

gegebenenfalls in Gegenwart eines Säureakzeptors und gegebenenfalls in Gegenwart eines Verdünnungsmittels umsetzt, oder

b) Phenoxyphenole der Formel

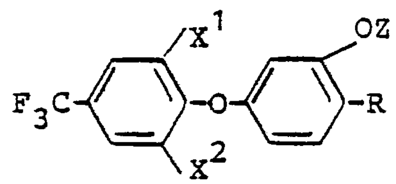

(IV)

in welcher

$X^1$, $X^2$ und R die oben angegebene Bedeutung haben und

Z für Wasserstoff, ein Alkalimetall oder ein Äquivalent eines Erdalkalimetalles steht,

mit Verbindungen der Formel

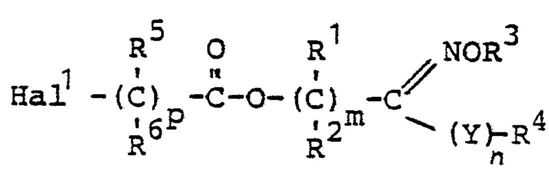

(V)

in welcher

$R^1$, $R^2$, $R^3$, $R^4$, $R^5$, $R^6$, Y, m, n und p die oben angegebene Bedeutung haben und

$Hal^1$ für Chlor oder Brom steht,

gegebenenfalls in Gegenwart eines Säureakzeptors und gegebenenfalls in Gegenwart eines Verdünnungsmittels umsetzt, oder

c) Trifluormethylphenoxy-phenyl-Derivate der Formel

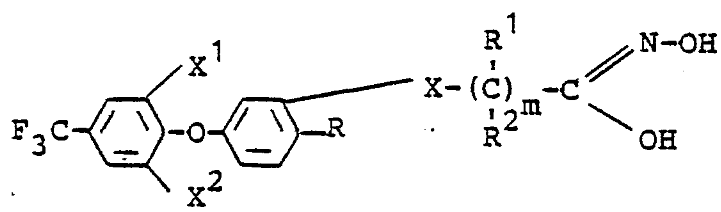

(Ia)

in welcher

$X^1$, $X^2$, X, R, $R^1$, $R^2$ und m die oben angegebene Bedeutung haben,

oder deren tautomere Verbindungen,

mit Dihalogenalkanen der Formel

$$Hal^2-(CH_2)_q-Hal^2 \quad (VI)$$

in welcher

q für die Zahlen 1, 2 oder 3 steht und

$Hal^2$ jeweils für Chlor oder Brom steht,

gegebenenfalls in Gegenwart eines Säureakzeptors, und gegebenenfalls in Gegenwart eines Verdünnungsmittels umsetzt, oder

d) Trifluormethylphenoxy-phenyl-Derivate der Formel

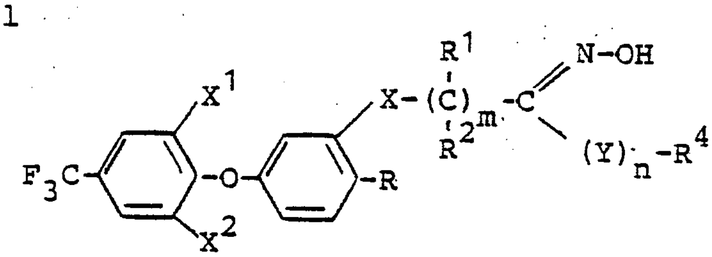

(Ib)

in welcher

$X^1$, $X^2$, X, R, $R^1$, $R^2$, $R^4$, Y, m und n die oben angegebene Bedeutung haben,

mit Alkylierungsmitteln der Formel

$$R^{3'}-Z^1 \qquad \text{(VII)}$$

in welcher

$R^{3'}$ für $C_1$-$C_5$-Alkyl steht und

$Z^1$ für Chlor, Brom, für eine p-Toluolsulfonyl-Gruppe oder für die Gruppierung $R^8$ $O-SO_2-O-$ steht, worin

$R^8$ für Wasserstoff oder $C_1-C_5$-Alkyl steht,

gegebenenfalls in Gegenwart eines Säureakzeptors und gegebenenfalls in Gegenwart eines Verdünnungsmittels umsetzt, oder

e) Halogenide der Formel

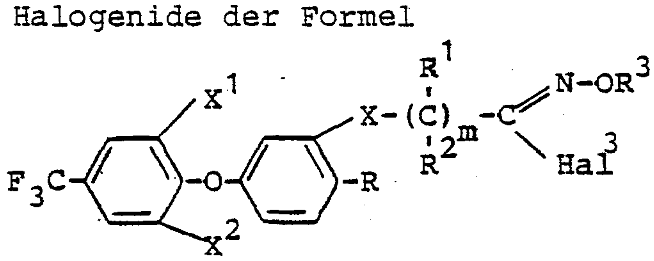

(VIII)

in welcher
$X^1, X^2, X, R, R^1, R^2, R^3$ und m die oben angegebene Bedeutung haben und

$Hal^3$ für Chlor oder Brom steht,

mit Verbindungen der Formel

$$R^4-Q-Z^2 \qquad \text{(IX)}$$

in welcher

$R^4$ die oben angegebene Bedeutung hat,

$Z^2$ für Wasserstoff, ein Alkalimetall oder ein Äquivalent eines Erdalkalimetalles steht, und

Q für Sauerstoff, Schwefel oder $NR^7$ steht, worin

$R^7$ für Wasserstoff oder $C_1$-$C_5$-Alkyl steht,

gegebenenfalls in Gegenwart eines Säureakzeptors und gegebenenfalls in Gegenwart eines Verdünnungsmittels umsetzt, oder

f) Nitrile der Formel

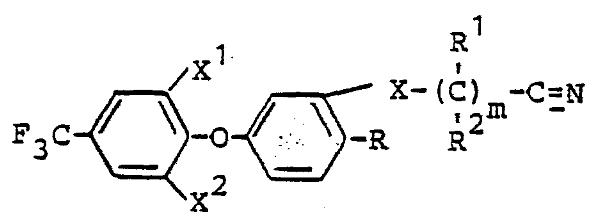

(X)

in welcher

$X^1$, $X^2$, X, R, $R^1$, $R^2$ und m die oben angegebene Bedeutung haben,

mit Hydroxylamin bzw. Hydroxylamin-Hydrochlorid oder -Hydrobromid

gegebenenfalls in Gegenwart eines Säureakzeptors und gegebenenfalls in Gegenwart eines Verdünnungsmittels umsetzt; oder

g) Verbindungen der Formel

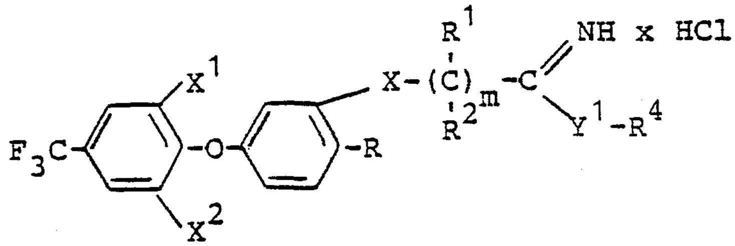

(XI)

in welcher

$X^1, X^2, X, R, R^1, R^2, R^4$, und m die oben angegebene Bedeutung haben und

$Y^1$ für Sauerstoff oder Schwefel steht,

mit Hydroxylamin bzw. Hydroxylamin-Hydrochlorid oder -Hydrobromid,

gegebenenfalls in Gegenwart eines Säureakzeptors und gegebenenfalls in Gegenwart eines Verdünnungsmittels umsetzt, oder

h) Trifluormethylphenoxy-phenyl-Derivate der Formel

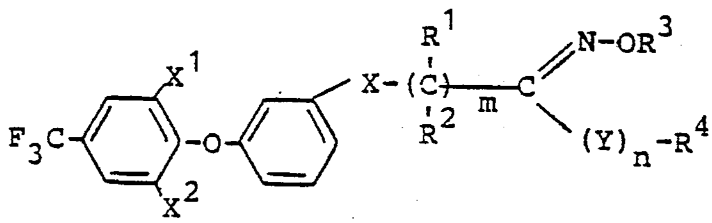

(Ic)

in welcher

$X, X^1, X^2, Y, R^1, R^2, R^3, R^4$, m und n die oben angegebene Bedeutung haben,

mit Salpetersäure, gegebenenfalls in Gegenwart eines Katalysators sowie gegebenenfalls in Gegenwart eines Verdünnungsmittels umsetzt, oder

i) Carbonyl-Derivate der Formel

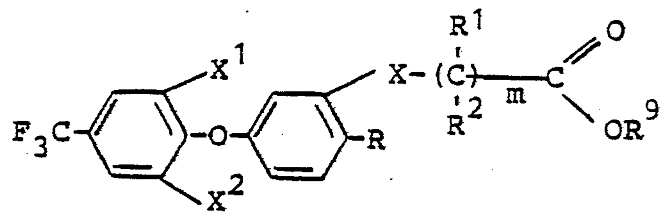

(XII)

in welcher
$X^1, X^2, X, R, R^1, R^2$ und m die oben angegebene Bedeutung haben und

$R^9$ für $C_1$-$C_5$-Alkyl steht,

mit Hydroxylamin oder Hydroxylamin-Hydrochlorid,

gegebenenfalls in Gegenwart eines Säureakzeptors und gegebenenfalls in Gegenwart eines Verdünnungsmittels umsetzt, oder

k) Trifluormethylbenzole der Formel

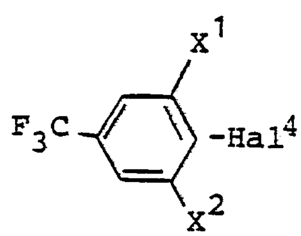

(XIII)

in welcher
$X^1$ und $X^2$ die oben angegebene Bedeutung haben und

$Hal^4$ für Chlor oder Brom steht,

mit Verbindungen der Formel

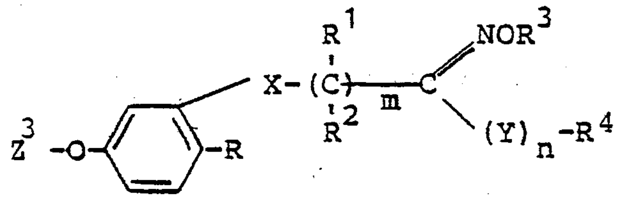

(XIV)

in welcher

X, Y, R, $R^1$, $R^2$, $R^3$, $R^4$, m und n die oben angegebene Bedeutung haben und

$Z^3$ für Wasserstoff, ein Alkalimetall oder ein Äquivalent eines Erdalkalimetalles steht,

gegebenenfalls in Gegenwart eines Säureakzeptors und gegebenenfalls in Gegenwart eines Verdünnungsmittels umsetzt, oder

l) Säurehalogenide der Formel

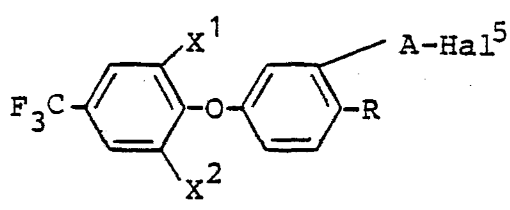

(XV)

in welcher
$X^1$, $X^2$ und R die oben angegebene Bedeutung haben,

$Hal^5$ für Chlor oder Brom steht und

A für eine -CO-Gruppe oder die Gruppierung der Formel

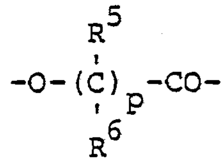

steht, in welcher

p, $R^5$ und $R^6$ die oben angegebene Bedeutung haben,

mit Verbindungen der Formel

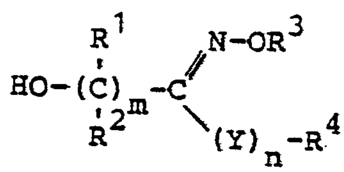

(XVI)

in welcher

$R^1$, $R^2$, $R^3$, $R^4$, Y, m und n die oben angegebene Bedeutung haben,

gegebenenfalls in Gegenwart eines Säureakzeptors und gegebenenfalls in Gegenwart eines Verdünnungsmittels umsetzt.

6. Herbizide Mittel, gekennzeichnet durch einen Gehalt an mindestens einem Trifluormethylphenoxy-phenyl-Derivat der Formel (I).

7. Verwendung von Trifluormethylphenoxy-phenyl-Derivaten der Formel (I) zur Bekämpfung von Unkräutern.

8. Verbindungen der Formel

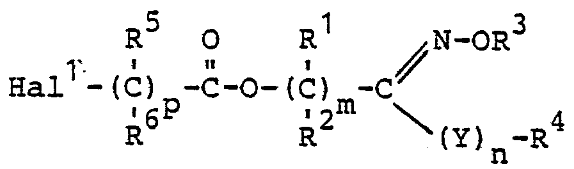

(V)

in welcher

$R^1$, $R^2$, $R^5$ und $R^6$ unabhängig voneinander für Wasserstoff oder $C_1$-$C_5$-Alkyl stehen,

$R^3$ für Wasserstoff oder $C_1$-$C_5$-Alkyl steht, oder

$R^2$ und $R^3$ gemeinsam für $C_1$-$C_3$-Alkandiyl stehen,

$R^4$ für Wasserstoff oder $C_1$-$C_5$-Alkyl steht, oder

$R^3$ und $R^4$ gemeinsam für $C_1$-$C_3$-Alkandiyl stehen,

Y für Sauerstoff, Schwefel, eine $-NR^7$-Gruppe oder eine CO-O-Gruppe steht,

$R^7$ für Wasserstoff oder $C_1$-$C_5$-Alkyl steht,

m für die Zahlen 1, 2 oder 3 steht,

n für die Zahlen 0 oder 1 steht,

p für die Zahlen 1, 2 oder 3 steht, und

$Hal^1$ für Chlor oder Brom steht.

9. Verfahren zur Herstellung von Verbindungen der Formel

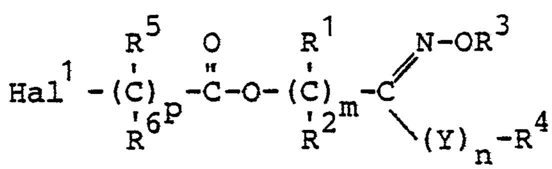

(V)

in welcher

$R^1$, $R^2$, $R^5$ und $R^6$ unabhängig voneinander für Wasserstoff oder $C_1$-$C_5$-Alkyl stehen,

$R^3$ für Wasserstoff oder $C_1$-$C_5$-Alkyl steht, oder

$R^2$ und $R^3$ gemeinsam für $C_1$-$C_3$-Alkandiyl stehen,

$R^4$ für Wasserstoff oder $C_1$-$C_5$-Alkyl steht, oder

$R^3$ und $R^4$ gemeinsam für $C_1$-$C_3$-Alkandiyl stehen,

Y für Sauerstoff, Schwefel, eine -$NR^7$-Gruppe oder eine CO-O-Gruppe steht,

$R^7$ für Wasserstoff oder $C_1$-$C_5$-Alkyl steht,

m für die Zahlen 1, 2 oder 3 steht,

n für die Zahlen 0 oder 1 steht,

p für die Zahlen 1, 2 oder 3 steht und

$Hal^1$ für Chlor oder Brom steht,

dadurch gekennzeichnet, daß man

α) Carbonylverbindungen der Formel

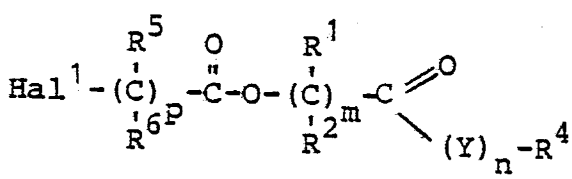

(XVIII)

in welcher

$R^1$, $R^2$, $R^4$, $R^5$, $R^6$, Y, $Hal^1$, m, n und p die oben angegebene Bedeutung haben,

mit Hydroxylamin bzw. mit Hydroxylamin-Derivaten in Gegenwart eines Verdünnungsmittels sowie gegebenenfalls in Gegenwart eines Säurebindemittels umsetzt, wobei das Hydroxylamin bzw. das Hydroxylamin-Derivat in Form eines Salzes eingesetzt werden kann,

oder

ß) Verbindungen der Formel

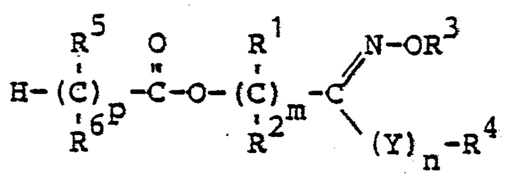

(XIX)

in welcher

$R^1, R^2, R^3, R^4, R^5, R^6$, Y, m, n und p die oben angebebene Bedeutung haben,

in üblicher Weise chloriert oder bromiert,

oder

γ) Alkohole der Formel

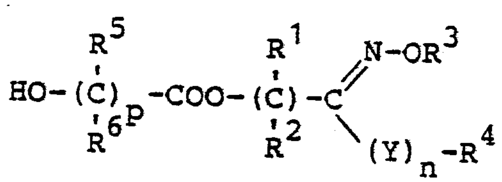

(XX)

in welcher

$R^1, R^2, R^3, R^4, R^5, R^6$, Y, m, n und p die oben angegebene Bedeutung haben,

mit Thionylchlorid oder Thionylbromid gegebenenfalls in Gegenwart eines Säureakzeptors

und gegebenenfalls in Gegenwart eines Verdünnungsmittels umsetzt,

oder

δ) Alkohole der Formel

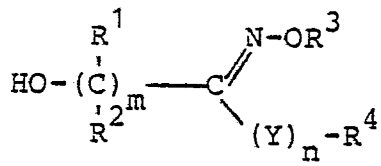

(XVI)

in welcher
$R^1$, $R^2$, $R^3$, $R^4$, Y, m und n die oben angegebene Bedeutung haben,

mit Carbonsäurehalogeniden der Formel

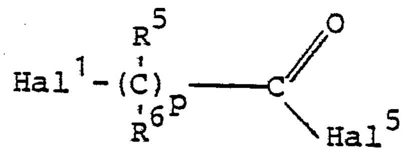

(XXI)

in welcher
$R^5$, $R^6$, $Hal^1$ und p die oben angegebene Bedeutung haben und
$Hal^5$ für Chlor oder Brom steht,

gegebenenfalls in Gegenwart eines Verdünnungsmittels und gegebenenfalls in Gegenwart eines Säureakzeptors umsetzt.

10. Halogenide der Formel

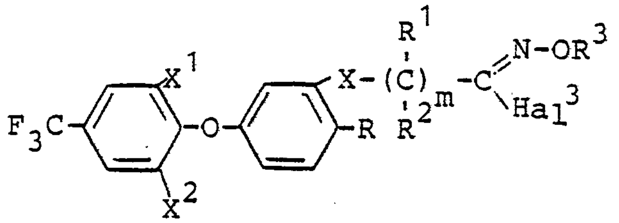

(VIII)

in welcher

$X^1$ für Wasserstoff, Fluor, Chlor, Brom, Jod, Nitro oder Cyano steht,

$X^2$ für Wasserstoff, Fluor, Chlor, Brom oder Jod steht,

R für Wasserstoff oder Nitro steht,

X für Sauerstoff, eine CO-O-Gruppe oder für die Gruppierung der Formel

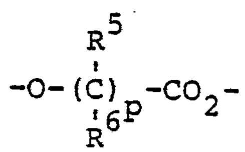

steht,

$R^1$, $R^2$, $R^5$ und $R^6$ unabhängig voneinander für Wasserstoff oder $C_1$-$C_5$-Alkyl stehen,

$R^3$ für Wasserstoff oder $C_1$-$C_5$-Alkyl steht, oder

$R^2$ und $R^3$ gemeinsam für $C_1$-$C_3$-Alkandiyl stehen,

m für die Zahlen 1, 2 oder 3 steht,

p für die Zahlen 1, 2 oder 3 steht und

$Hal^3$ für Chlor oder Brom steht.

11. Verfahren zur Herstellung von Halogeniden der Formel

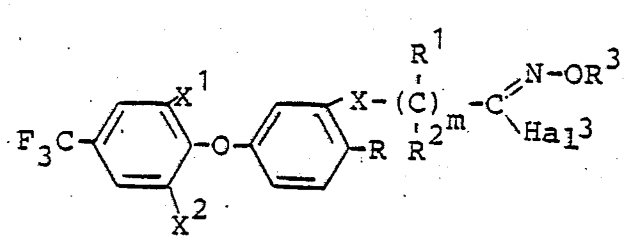 (VIII)

in welcher

$X^1$ für Wasserstoff, Fluor, Chlor, Brom, Jod, Nitro oder Cyano steht,

$X^2$ für Wasserstoff, Fluor, Chlor, Brom oder Iod steht,

R für Wasserstoff oder Nitro steht,

X für Sauerstoff, eine CO-O-Gruppe oder für die Gruppierung der Formel

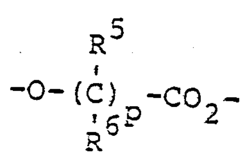

steht,

$R^1$, $R^2$, $R^5$ und $R^6$ unabhängig voneinander für Wasserstoff oder $C_1$-$C_5$-Alkyl stehen,

$R^3$ für Wasserstoff oder $C_1$-$C_5$-Alkyl steht, oder

$R^2$ und $R^3$ gemeinsam für $C_1$-$C_3$-Alkandiyl stehen,

m für die Zahlen 1, 2 oder 3 steht,

p für die Zahlen 1, 2 oder 3 und

$Hal^3$ für Chlor oder Brom steht,

dadurch gekennzeichnet, daß man Hydroxamsäure-Derivate der Formel

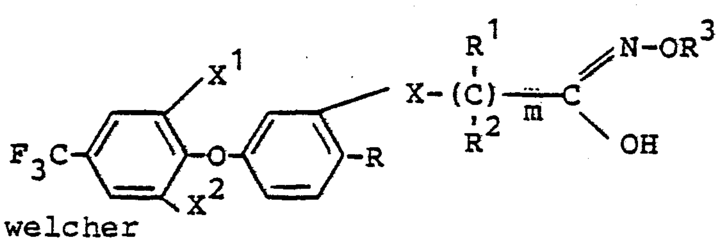

(Ih)

in welcher

$X^1$,$X^2$,X,R,$R^1$,$R^2$,$R^3$ und m die oben angegebene Bedeutung haben, bzw. deren tautomere Verbindungen, oder

Aldoxime der Formel

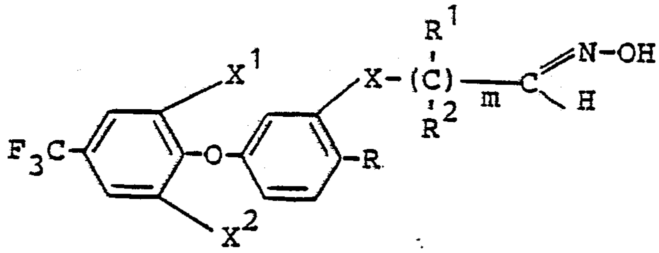

(Ii)

in welcher

$X^1$, $X^2$, X, R, $R^1$, $R^2$ und m die oben angegebene Bedeutung haben,

mit Chlorierungs- oder Bromierungsmitteln, gegebenenfalls in Gegenwart eines Säureakzeptors sowie gegebenenfalls in Gegenwart eines Verdünnungsmittels umsetzt.

12. Verbindungen der Formel

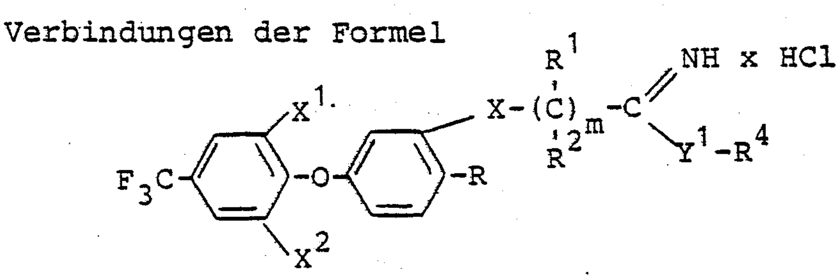

(XI)

in welcher

$X^1$ für Wasserstoff, Fluor, Chlor, Brom, Jod, Nitro oder Cyano steht,

$X^2$ für Wasserstoff, Fluor, Chlor, Brom oder Jod steht,

R für Wasserstoff oder Nitro steht,

X für Sauerstoff, eine CO-O-Gruppe oder für die Gruppierung der Formel

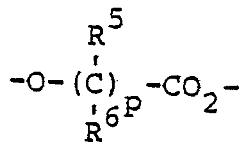

steht,

$R^1$, $R^2$, $R^5$ und $R^6$ unabhängig voneiander für Wasserstoff oder $C_1$-$C_5$-Alkyl stehen,

$R^4$ für Wasserstoff oder $C_1$-$C_5$-Alkyl steht,

$Y^1$ für Sauerstoff oder Schwefel steht,

m für die Zahlen 1, 2 oder 3 steht,

p für die Zahlen 1, 2 oder 3 steht.

13. Verfahren zur Herstellung von Verbindungen der Formel

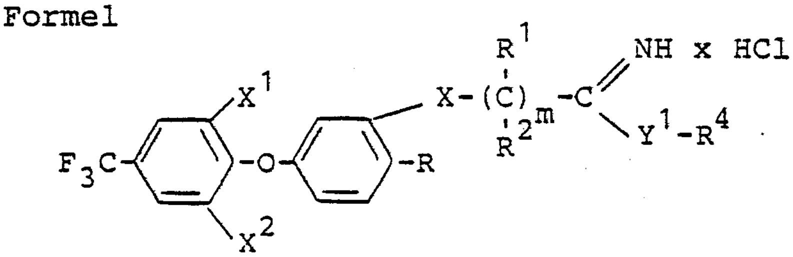

(XI)

in welcher

$X^1$ für Wasserstoff, Fluor, Chlor, Brom, Jod, Nitro oder Cyano steht,

$X^2$ für Wasserstoff, Fluor, Chlor, Brom oder Jod steht,

R für Wasserstoff oder Nitro steht,

X für Sauerstoff, eine CO-O-Gruppe oder für die Gruppierung der Formel

$$-O-(\underset{R^6}{\overset{R^5}{C}})_p-CO_2-$$

steht,

$R^1$, $R^2$, $R^5$ und $R^6$ unabhängig voneinander für Wasserstoff oder $C_1$-$C_5$-Alkyl stehen,

$R^4$ für Wasserstoff oder $C_1$-$C_5$-Alkyl steht,

$Y^1$ für Sauerstoff oder Schwefel steht,

m für die Zahlen 1, 2 oder 3 steht und

p für die Zahlen 1, 2 oder 3 steht,

dadurch gekennzeichnet, daß man Nitrile der Formel

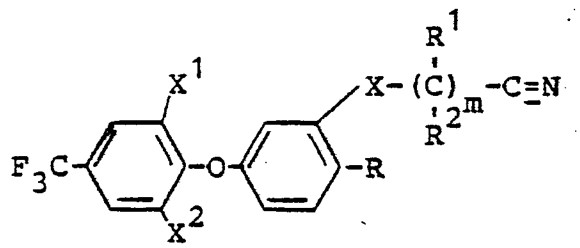

(X)

in welcher

$X^1$, $X^2$, X, $R^1$, $R^2$, R und m die oben angegebene Bedeutung haben,

mit Verbindungen der Formel

$$R^4-Y^1-H \qquad (XXII)$$

in welcher $R^4$ und $Y^1$ die oben angegebene Bedeutung haben,

in Gegenwart von Chlorwasserstoff und in Gegenwart eines Lösungsmittels umsetzt.

Europäisches Patentamt

**EUROPÄISCHER RECHERCHENBERICHT**

0095683 Nummer der Anmeldung

EP 83 10 5032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 013 660 (CIBA-GEIGY AG) * Beispiele 19, 22 * --- | 1-4,6, 7 | C 07 C 131/105<br>C 07 D 273/01<br>C 07 C 131/00<br>A 01 N 37/52<br>A 01 N 35/10<br>C 07 C 161/00 //<br>C 07 D 265/02<br>C 07 C 119/16<br>C 07 C 135/00 |
| A | EP-A-0 034 012 (MOBIL OIL CORP.) * Anspruch 1 * --- | 1-4,6, 7 | |
| A | EP-A-0 002 246 (HOFFMANN-LA ROCHE & CO. AG) * Zusammenfassung * --- | 1-4,6, 7 | |
| A | DE-A-3 017 795 (PPG INDUSTRIES) * Anspruch 1 * ----- | 1-4,6, 7 | |
| | | | **RECHERCHIERTE SACHGEBIETE (Int. Cl. 3)** |
| | | | A 01 N 35/10<br>C 07 C 119/16<br>C 07 C 131/00<br>C 07 C 135/00<br>C 07 C 161/00<br>C 07 D 273/01 |

Der vorliegende Recherchenbericht wurde für alle Patentansprüche erstellt.

| Recherchenort | Abschlußdatum der Recherche | Prüfer |
|---|---|---|
| BERLIN | 22-07-1983 | BREW C.H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82